# EUROPEAN PATENT SPECIFICATION

(11) **EP 0 766 578 B1**
(45) Date of publication and mention of the grant of the patent: **04.10.2000**
(21) Application number: 95925261.0
(22) Date of filing: 23.06.1995
(51) Int. Cl.: A61N 1/30, A61B 5/00, A61B 10/00

(54) **IONTOPHORETIC SAMPLING DEVICE**
EINRICHTUNG ZUR IONTOPHORETISCHEN PROBENNAHME
DISPOSITIF POUR LE PRELEVEMENT D'ECHANTILLONS PAR IONTOPHORESE

(30) Priority: 24.06.1994 US 265048; 10.01.1995 US 373931
(43) Date of publication of application: 09.04.1997
(60) Divisional application: 00200524.7
(73) Proprietor: CYGNUS, INC., Redwood City, CA 94063 (US)
(72) Inventor: TAMADA, Janet, Belmont, CA 94002 (US); AZIMI, Nooshin T., Redwood City, CA 94065 (US); LEUNG, Lewis, San Carlos, CA 94070 (US); LEE, Richard K-T, Fremont, CA 94555 (US); PLANTE, Phillip J., Sunnyvale, CA 94086 (US); BHAYANI, Bhaskar V., Fremont, CA 94539 (US); CAO, Michael, Sunnyvale, CA 94086 (US); TIERNEY, Michael J., San Jose, CA 95112 (US); VIJAYAKUMAR, Prema, Fremont, CA 94539 (US)
(74) Representative: Hallybone, Huw George
(86) International application number: US9507692
(87) International publication number: WO9600110

(56) References cited:
- EP-A- 0 483 883
- US-A- 4 708 716
- US-A- 5 013 293
- US-A- 5 036 861
- US-A- 5 069 908
- US-A- 5 279 543

## Description

### BACKGROUND OF THE INVENTION

This invention relates generally to the transdermal monitoring of the concentration of substances in blood and, in particular, to the use of reverse iontophoresis or electroosmosis for the continuous transdermal monitoring of blood glucose.

Many diagnostic tests are performed on humans by evaluating the amount or existence of substances in the blood. One blood substance of interest is blood glucose. Typically, blood samples are removed from a subject by either using a syringe or by pricking the skin. The amount of blood drawn, of course, depends upon the amount of blood required for testing. Thereafter, the blood sample may be prepared and specifically tested for a variety of substances using techniques well known in the art.

Over the last few years methods of determining the concentration of blood glucose or other substances without drawing blood have been developed. For example, Stanley U.S. Patent No. 5,139,023 describes a transdermal glucose monitoring apparatus that uses a permeability enhancer, such as a natural bile salt, to facilitate transdermal movement of glucose along the concentration gradient between the higher glucose concentration in the interstitial fluid and the lower glucose concentration in the receiving medium. In some embodiments, the receiving medium is the aqueous portion of a hydrogel support adhered to the subject's skin. Stanley measured the glucose concentration within the hydrogel by removing the hydrogel from the subject's skin, placing the hydrogel in water and letting the glucose diffuse out of the hydrogel into the water. The water was then analyzed for glucose concentration.

Sembrowich U.S. Patent No. 5,036,861 describes a glucose monitor that collects the subject's sweat through a skin patch attached to the subject's wrist. Sembrowich describes the use of iontophoresis to transdermally introduce a gel into the subject's skin. The gel contains a cholinergic agent for stimulating the secretion mechanism of the eccrine sweat gland and agents that minimize or prevent loss of glucose from the sweat as it travels from the sweat gland to the skin patch. The Sembrowich device uses electrodes to measure the glucose level in the collected sweat by some unspecified method.

Schoendorfer U.S. Patent No. 5,076,273 describes a method and apparatus for determination of chemical species in body fluid. Sweat expressed from the subject's skin is collected in a patch adhered to the subject's skin. The patch concentrates the sweat in a binder layer by driving off a portion of the collected water. The collected analyte binds with a specific binding partner in the patch to present a visual indication of its presence in the patch.

Schroeder U.S. Patent No. 5,140,985 discloses a sweat-collecting device mounted on a subject. The device has an electrode-based glucose detection system that can give a qualitative indication of blood glucose concentration.

Glikfeld U.S. Patent No. 5,279,543 describes the use of iontophoresis to sample a substance through skin into a receptor chamber on the skin surface. In one embodiment, Glikfeld describes an in vitro device consisting of two gel electrodes attached to one side of hairless mouse skin. Radiolabeled glucose is placed on the other side of the skin, and current is applied to the electrodes for a period of time. Since the device does not comprise sensor means, the electrodes are then analyzed for radioactivity content by conventional liquid scintillation counting. (See Glikfeld col. 7, line 51, to col. 8, line 1.) Glikfeld suggests that this sampling procedure can be combined with a specific glucose biosensor or glucose selective electrodes, but Glikfeld does not describe how that combination might be achieved. (See Glikfeld col. 9, lines 27-34.) U.S. Patent No. 4,708,716 describes a transdermal drug delivery system with a passive sensor electrode to monitor the amount of drug delivered. The sensor electrode is insulated from the drug delivering electrode(s), but all electrodes are simultaneously part of the same circuit, thus providing for simultaneous detection and delivery. U.S. Patent No. 5,013,293 also describes a transdermal drug delivery system, however, sensor means are not mentioned.

### SUMMARY OF THE INVENTION

This invention improves on prior art transdermal monitoring devices by providing an iontophoretic sampling device with an integrated sensor for monitoring the blood concentration of target substances or constituents noninvasively.

A general object of this invention is to provide a means of extracting substances in the blood through the skin using reverse iontophoresis or electroosmosis. In a preferred embodiment, one or more collection reservoirs are held in contact with the subject's skin. The reservoirs contain a conductive medium and are in turn in contact with electrodes and a means for providing electric potential or current between the collection reservoir site and another site on the subject's skin. The collection reservoirs are also in contact with a means for sensing and/or quantifying the concentration of a target substance.

This invention is particularly advantageous over drawing blood from a subject when extensive time-consuming sample preparation is required to perform a diagnostic test on whole blood. This invention is also advantageous for neonates (who have a small blood volume) and for subjects who must have frequent testing.

In a preferred embodiment, the collection reservoirs consist of an ionically conductive hydrogel or wicking material soaked with an ionically conductive medium. The advantage of using a hydrogel or wicking material as the extraction means is that by collecting the target substances into a semi-solid medium which is in contact with a target substance sensor, there is direct feedback to the subject of whether, or how much of, the target substance is in the blood. Also, because the target substances are extracted into a hydrogel or other medium of small volume, the concentration of the substance is higher than can be obtained by reverse iontophoretic extraction into a liquid solution, as in some prior art devices.

There are a number of different substances that can be sampled using the reverse iontophoresis/electroosmosis techniques described herein. Charged (i.e., having a negative or positive ionic charge) substances will be extracted in the highest concentration. Uncharged substances will be extracted at lower, but still quantifiable, concentrations. An example of an uncharged substance is glucose. Obviously, there are many other substances in the body which would be of interest to sample and analyze. These include, but are not limited to, amino acids, enzyme substrates or products indicating a disease state or condition, other markers of disease states or conditions, drugs of abuse, therapeutic drugs, and electrolytes.

Different substances will be extracted at each electrode, as one electrode is positively charged and will attract negatively charged substances, and the other electrode is negatively charged and will attract positively charged substances. Uncharged substances may be extracted at either electrode, although they are typically extracted at higher concentrations at the negatively charged electrode. Each ionically conductive medium in contact with the electrodes, therefore, may be analyzed for different substances.

The invention is particularly (although not exclusively) adapted to a method for continuous *in vivo* monitoring of the blood glucose level of a patient comprising the following steps:
(a) placing a collection reservoir on a collection site on a tissue surface of the patient;
(b) applying electrical energy to the collection site to move glucose or a glucose metabolite into the collection reservoir;
(c) analyzing the collection reservoir for concentration of glucose or glucose metabolite;
(d) correlating the concentration determined in step (c) with blood glucose level; and
(e) performing steps (a)-(d) substantially continuously.

The method correlates measured glucose levels with blood glucose levels over an extended period of time and tracks changes in blood glucose levels.

The invention is also particularly, but not exclusively, directed to a glucose monitor comprising:
first and second glucose collection reservoirs each containing glucose collection medium selected from a group consisting of water and saline solution;
a power supply having a positive connector and a negative connector;
conductors and a switch electrically connecting the first and second collection reservoirs to the power supply positive connector and the power supply negative connector, the switch having a first position in which the first collection reservoir is electrically connected to the positive connector and the second collection reservoir is electrically connected to the negative connector and a second position in which the second collection reservoir is electrically connected to the positive connector and the first collection reservoir is electrically connected to the negative connector. Preferably, the glucose monitor is one wherein the first and second collection reservoirs each further comprise an electrode in electrical communication with the collection medium and with the power supply through a conductor and the switch.

The invention is described in more detail below with reference to the drawings.

### BRIEF DESCRIPTION OF THE DRAWINGS

Figure 1A is a block diagram of a glucose monitoring device according to this invention.

Figure 1B shows a glucose monitoring device useful for practicing this invention in place on a patient.

Figure 1C is an exploded drawing of a collection reservoir assembly.

Figure 1D is a cross-sectional drawing of the electrode/collection reservoir assembly of Figure 1C.

Figure 2 is a graph of the results of a blood glucose monitoring of a fasting subject (1) using a standard protocol.

Figure 3 is a graph of the results of a blood glucose monitoring of a fasting subject (2) using a standard protocol.

Figure 4 is a graph of the results of a blood oral glucose tolerance monitoring of subject (1) as Figure 2 using a standard protocol.

Figure 5 is a graph of the results of a blood oral glucose tolerance monitoring of subject (1) as Figure 4 showing only the cathode.

Figure 6 is a graph of the results of a blood oral glucose tolerance monitoring of a subject (1) as Figure 5 at the cathode showing a 40 min lag time.

Figure 7 is a graph of the results of a blood glucose monitoring of a fasting subject (1) as in Figure 2 showing a drift in flux at the cathode.

Figure 8 is a graph of the results of a blood glucose monitoring of a fasting subject (1) as in Figure 7 showing a linear fit to the drift at the cathode in flux over 3 h.

Figure 9 is a graph of the results of a blood oral glucose tolerance monitoring of a subject (1) as in Figure 4 showing subtraction of linear drift as in Figure 8 from cathode flux.

Figure 10 is a graph of the results of a blood oral glucose tolerance monitoring of a subject (1) as in Figure 9,showing extracted glucose correlates with blood glucose with a 40 minute lag as in Figure 6.

Figure 11 is a graph of the results of an oral glucose tolerance monitoring of a subject (3) showing correlation at the anode using a standard protocol.

Figure 12 is a graph of the results of an oral glucose tolerance monitoring of a subject (3) as in Figure 11 showing a 20 min time lag.

Figure 13 is a graph of the results of an oral glucose tolerance monitoring of a subject (4) showing higher flux and better correlation at the anode using a standard protocol.

Figure 14 is a graph of the results of an oral glucose tolerance monitoring of a subject (4) as in Figure 13 showing a 20 min time lag.

Figure 15 is a graph of the results of an oral glucose tolerance monitoring of a subject (5) showing correlation at both the cathode and anode using a standard protocol.

Figure 16 is a graph of the results of an oral glucose tolerance monitoring of a subject (5) as Figure 15 showing a 20 min time lag.

Figure 17 is a graph of the results of a blood oral glucose tolerance monitoring experiment on subject (6), right arm, using a standard protocol.

Figure 18 is a graph of the results of a blood oral glucose tolerance monitoring experiment on subject (6), left arm, using an alternating protocol.

Figure 19 is a graph of the results of a blood oral glucose tolerance monitoring experiment on subject (7), right arm, using a standard protocol.

Figure 20 is a graph of the results of a blood oral glucose tolerance monitoring experiment on subject (7), left arm, using an alternating protocol.

Figure 21 is a graph of the results of a fasting blood glucose monitoring experiment on subject (6) using an alternating protocol.

Figure 22 is a graph of the results of a fasting blood glucose monitoring experiment on subject (6) as Figure 21 showing only the cathode.

Figure 23 is a graph of the results of a blood glucose monitoring of the same subject (6) as Figure 18, left arm, but with the drift adjusted for the cathode as in Figure 22.

Figure 24 is a graph of the results of a blood glucose monitoring of the same subject (6) as Figure 18 but with the time lag and drift adjusted for the cathode.

Figure 25 is a graph of the results of a blood glucose monitoring of the same subject (6) as Figure 18 but with the drift adjusted for the anode as in Figure 21.

Figure 26 is a graph of the results of a blood glucose monitoring of the same subject (6) as Figure 18 but with the time lag and drift adjusted for the anode.

Figure 27 is a graph of the results of a blood glucose monitoring of the same subject (7) as Figure 20, left arm, but with the time lag and drift adjusted for the cathode.

Figure 28 is a graph of the results of a blood glucose monitoring of the same subject (7) as Figure 20, left arm, but with the time lag and drift adjusted for the anode.

Figure 29 is a graph of the results of a blood glucose monitoring experiment using several cycles of direct current and several cycles of alternating polarity.

Figure 30 is a graph of the results of iontophoretic sampling of glucose compared to passive and wash controls.

Figure 31 is a graph of the cumulative amount of glucose extracted passively from the skin plotted as a function of the square-root of time. Each line corresponds to a single subject.

Figure 32 is a graph of the results of glucose sampling at pH 8.2 using the standard method.

Figure 33 is a graph of the results of glucose sampling at pH 8.2 using alternating polarity.

Figure 34A is a top view of an embodiment of the invention featuring a co-planar iontophoresis electrode and sensor electrodes.

Figure 34B is a cross-sectional side view of the embodiment of the invention shown in Figure 34A.

Figure 35A is a top view of another embodiment of the invention in which the sensor electrodes are located on top of the iontophoresis electrode.

Figure 35B is a cross-sectional side view of the embodiment of the invention shown in Figure 35A.

Figure 36A is a top view of an embodiment of the invention in which the sensor electrodes are located beneath the iontophoresis electrode.

Figure 36B is a side view of the embodiment of the invention shown in Figure 36A.

Figure 37A is a top view of an embodiment of the invention featuring an annular iontophoresis electrode surrounding the sensor electrodes.

Figure 37B is a side view of the embodiment shown in Figure 37A.

Figure 38 is an exploded view of the embodiment of Figure 37 and a strap-on housing.

Figure 39 is a side view showing an optical sensing scheme.

Figure 40 is a schematic diagram showing an iontophoretic collection system according to this invention.

Figure 41 is an exploded view of one embodiment of an iontophoretic collection system according to this invention.

### DETAILED DESCRIPTION OF THE PREFERRED EMBODIMENT

### Definitions

Unless defined otherwise all technical and scientific terms used herein have the same meaning as commonly understood by one of ordinary skill in art to which this invention is directed. Although any methods and materials similar or equivalent to those described herein can be used in the practice or testing of this invention, the preferred methods and materials are now described.

The following terms are used throughout the application:

By "applying electrical energy to the collection site to move glucose or a glucose metabolite into the collection reservoir" is meant applying electrical energy of sufficient strength and duration to the tissue surface of a patient in order to transport the glucose or a glucose metabolite from beneath the tissue at a collection site on the surface of the tissue into a defined collection area. This includes the method known as iontophoresis.

"Iontophoresis" as used herein means a method for transporting positive or negative ions or even uncharged non-ionic substances across tissue through the application of electrical energy to the tissue. In conventional iontophoresis, a reservoir is provided at the tisspe surface to serve as a source of material to be transported or as a collector of material transported.

"Reverse iontophoresis" refers to the movement of an ionically charged substance from the blood across the epithelial membrane and into a collection device under the influence of an applied electric potential. "Electroosmosis" refers to the movement-of a charged or uncharged substance through a membrane into a collection device under the influence of an applied electric potential. "Reverse iontophoresis." and "electroosmosis" will be used interchangeably herein to refer to the movement of an ionically charged or uncharged substance across an epithelial membrane upon the application of an electric potential to the epithelial membrane through an ionically conductive medium.

As used herein "sampling" or "monitoring" means the extraction of a substance from an organism through the tissue. The tissue or skin can be natural or artificial tissue and can be of plant or animal nature, such as natural or artificial skin, blood vessel tissue, intestinal tissue and the like. The term "artificial" as used herein means an aggregation of cells of monolayer thickness or greater which are grown or cultured in vivo or in vitro and which function as a tissue of an organism but are not actually derived, or excised, from a pre-existing source or host. The subject/patient or host organism can include warm-blooded animals and is particularly, a mammal, such as rats, mice, pigs, dogs, cats, cattle, sheep, horses and especially a human being. When the tissue is human skin, the tissue surface is the stratum corneum surface or mucosal tissue, such as in the oral, nasal or vaginal cavity.

"Epithelial layer" refers to a membrane surface, such as skin and mucosal membranes.

### General Materials and Methods

The apparatus of this invention may be used to transdermally sample and quantify or qualify the level of a substance in a patient's blood. The apparatus will be initially described as used to transdermally sample and quantify or qualify glucose or a glucose metabolite. It should be understood that the apparatus of this invention may be used to transdermally sample and quantify or qualify other substances as well.

There is a need to sample and quantify or qualify glucose or a glucose metabolite in the blood, for example, for the purpose of diabetes diagnosis and treatment. This preferably needs to be done without the invasive withdrawal of blood by a needle into a collection reservoir/container. For maximum benefit, the sampling and quantification also need to be performed substantially continuously and the measured glucose values need to track actual blood glucose levels.

Accordingly, the apparatus of the invention is useful in continuously monitoring the levels of glucose or a glucose metabolite from the body. The apparatus can also be used for the measurement of blood glucose levels in either a semi-continuous or a single measurement method. The apparatus can provide electrodes or other means for applying electric current to the tissue at the collection site; one or more collection reservoirs or sampling chambers to receive the glucose; and a glucose concentration measurement system. A preferred embodiment of the apparatus is described in more detail below.

In order to use the apparatus of this invention, a glucose collection reservoir is placed at a collection site on a tissue surface of the patient, for example, on the stratum corneum of the patient's skin or on the mucosal epithelium. Electrical energy is applied to the tissue at the collection site to move glucose or a glucose metabolite from the tissue into the collection reservoir. The collection reservoir is analyzed periodically to measure the glucose or glucose metabolite concentration therein, and this measured concentration value is correlated with the patient's blood glucose level. These steps are repeated to monitor the patient's blood glucose level substantially continuously and to track changes in the patient's blood glucose level.

In a preferred procedure, two electrodes provide the electrical energy to the tissue at the collection site or sites. Two collection reservoirs are provided, one at or near each electrode. The applied electrical current is preferably in the range of about 0.01 to about 2 mA/cm². In applying the electrical energy, the polarity of the electrodes is alternated at a rate in the range of about 0.1 Hz (one switch every 10 seconds) to about 1 switch every hour, so that each electrode is alternately a cathode or an anode. Glucose is collected in both reservoirs, but is most preferably measured in the reservoir acting as the cathode. In other words, the reservoir in which glucose concentration is monitored alternates as the polarity of the electrodes alternates.

The reversal of polarity during the glucose sampling when using the apparatus of the invention has advantages, including: (1) an unexpected enhancement of the correlation between blood and iontophoretically extracted samples under the alternating polarity protocol as compared to a non-alternating polarity protocol, (2) a particularly unexpected increase in the normalized flux rate, and (3) the avoidance of depletion of the AgCl plating on AgCl electrodes or pH variations which occur for non-alternating systems.

The polarity switching can be manual or automatic. The polarity switching can be of any frequency, especially at frequencies between about 1 cycle per 20 seconds to about 1 cycle per 4 hours, preferably between about 1 cycle per 20 seconds to about 1 cycle per 2 hours, more preferably between about 1 cycle per minute to about 1 cycle per 2 hours, or between about 1 cycle per 10 minutes to about 1 cycle per hour, and especially about 1 cycle per half hour. By cycle is meant one interval of current in each direction. The application of electrical energy in a given cycle may cease during glucose analysis of the collection medium.

In the preferred procedure, part or all of the glucose collection medium is extracted from the collection reservoir and analyzed for glucose concentration. One preferred analysis method is high pressure liquid chromatography (HPLC), although other analysis methods may be used.

To provide useful information regarding blood glucose levels, the glucose concentrations detected in the collection medium must be correlated with the patient's blood glucose. Collection medium glucose is computed in flux terms, such as nmoles/cm²•hr. This information must be translated into blood glucose values (expressed, *e.g.*, in terms of mg/dl) that correspond to the measured glucose flux.

The correlation between blood glucose concentration and measure glucose flux may be expressed in terms of four variables: the time lag between blood glucose concentration values and measured glucose flux values; linear drift over time of the measured glucose flux values; and the slope and intercept (*i.e.*, the low-end blood glucose value below which no glucose flux is detected) of a line relating time lag and drift-corrected glucose flux data to blood glucose concentration. Correlation between measured glucose flux and actual blood glucose concentration is accomplished through a calibration procedure which relates blood glucose data and glucose flux data.

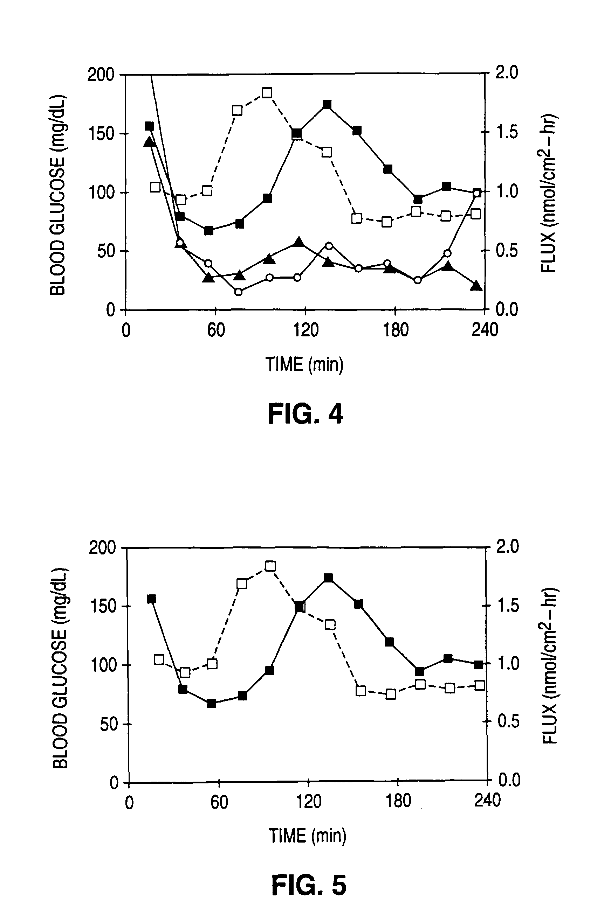

Time lag is defined as the constant time shift between blood glucose and glucose flux curves that achieves a best fit in curve shape along the time axis within predefined tolerances. The amount of time shift may be determined manually or automatically with the aid of a computer or other processor using known curve shape comparison algorithms. Time shift is illustrated in Figs. 5 & 6; Figs. 9 & 10; Figs. 11 & 12; Figs. 13 & 14; Figs. 15 & 16; Figs. 23 & 24; and Figs. 25 & 26.

Drift is defined as the linear change with time of the measured glucose flux for a given blood glucose concentration. Drift is computed by shifting the plotted glucose flux curve with respect to the blood glucose concentration curve along the glucose flux axis by a constant multiple of the time variable to achieve a best fit. The value of the constant multiple may be computed in many different ways. For example, experimental data taken from a given subject at given times under "baseline" conditions (*i.e.*, the patient is fasting and the blood glucose level is therefore constant) may be subtracted from measured glucose flux data point by point during the oral glucose tolerance test at the same times for the same patient.

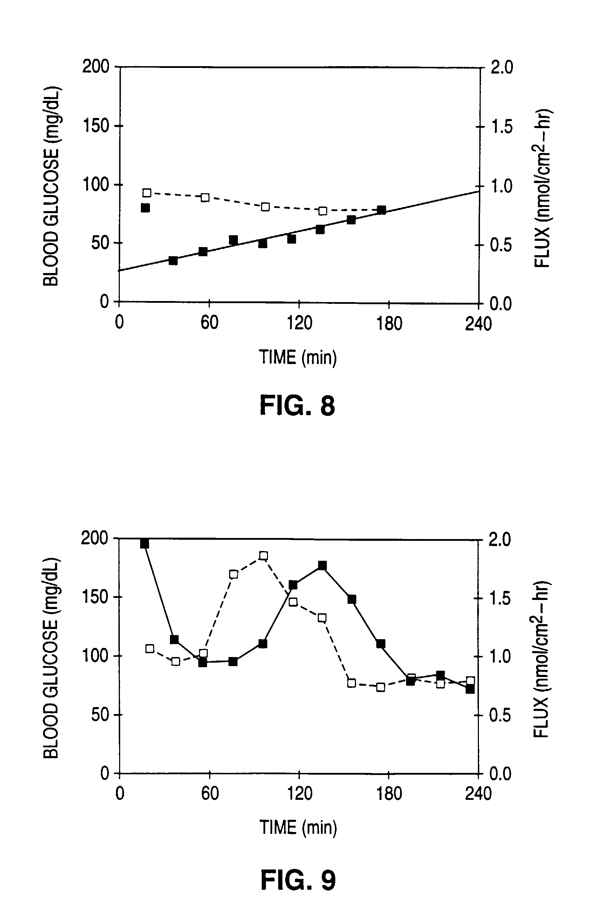

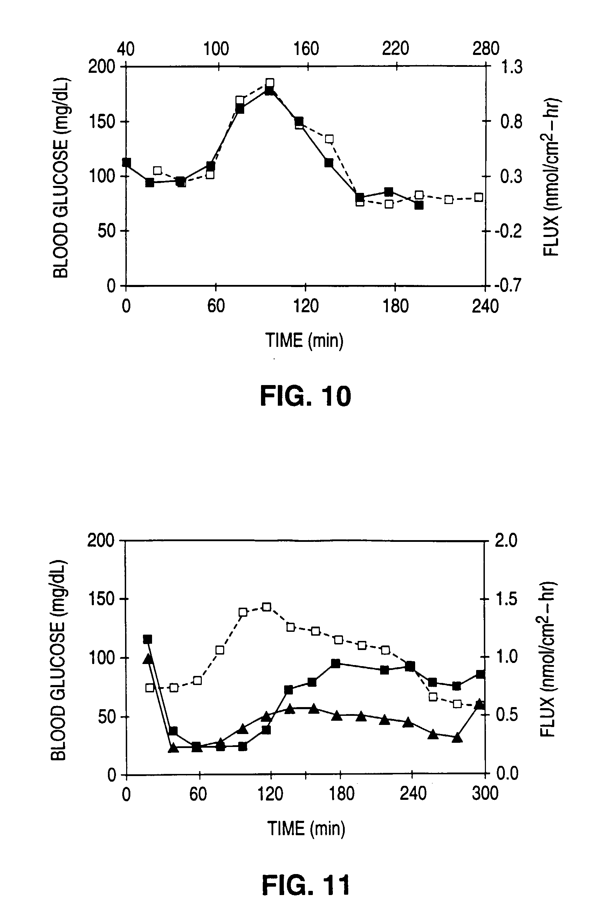

As another example, a linear fit (obtained, *e.g.*, from a least squares linear regression) of the baseline data for a given patient may be subtracted from experimental data obtained during the oral glucose tolerance test. This approach is illustrated in Figure 8 (baseline data), Figure 5 (experimental data without drift adjustment), Figure 9 (drift-adjusted experimental data) and Figure 10 (time lag and drift-adjusted experimental data).

Measured glucose flux values can also be corrected without first obtaining baseline values. One approach is to estimate the value of drift time constant, adjust for drift based on the estimate, and determine the correlation between drift-corrected measured values and measured blood glucose values. The estimated drift time constant can then be increased or decreased, and the correlation recalculated, until an acceptable tolerance between measure glucose flux values and blood glucose values is achieved.

The drift time constant may also be computed by measuring a different physiological parameter, such as the electrical resistance of the patient's skin (which may change with time in the same manner as the glucose flux value drift) or a parameter that remains relatively constant over time, such as the pH of the patient's blood.

Finally, the slope and intercept of the relationship between the time and drift corrected values is computed using a linear best fit of the data. These steps may be performed by hand or by a suitably programmed processor or computer.

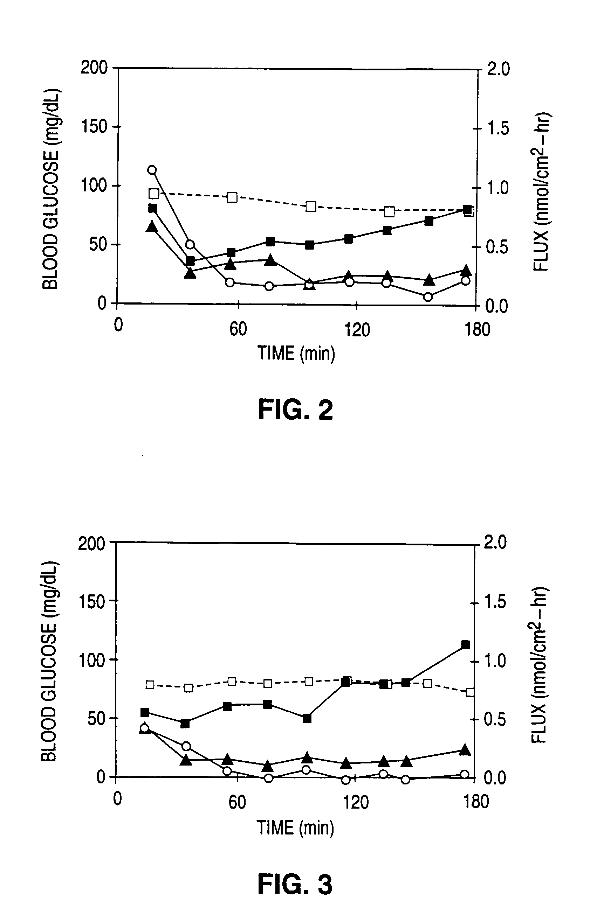

A certain amount of non-systemic glucose may be present in the stratum corneum prior to application of the glucose monitoring device as shown in Figures 2, 3 and 21. For using the preferred embodiment, therefore, a time delay is provided between the start of the sampling and the time of analysis. This time delay may be on the order of 1 hour.

The apparatus of this invention may be used substantially continuously for an indefinite period of time. The use of the invention may also include the step of treating the patient in response to the determined (*i.e.,* correlated) blood glucose level.

When the device is operated by alternating polarity, the collection reservoir/sampling chamber of the device of the invention can be in the form of (1) a single chamber with an electrode, which alternates polarity, with an indifferent counterelectrode which also alternates polarity, with the electrode in the sampling chamber acting as anode, cathode or both during the course of sampling, (2) two chambers in which the anode and cathode chambers are on alternate sites as the polarity alternates or (3) two or more chambers in which the polarity alternates during the treatment allowing for signal averaging between cathode and anode.

The collection reservoir/sampling chamber for receiving the sample of the target substance from the subject can be in the form of an adhesive patch enclosing the electrode means. A wide variety of adhesives for patches are known in the iontophoresis art.

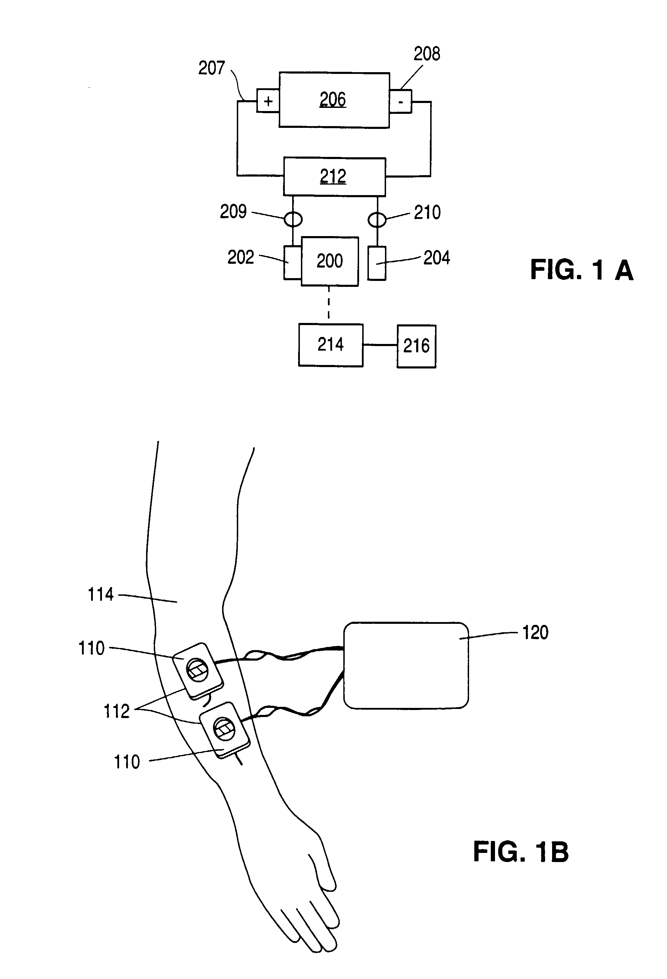

Figure 1A shows in block diagram form a continuous glucose monitor according to this invention. The monitor includes a collection reservoir 200 containing a glucose collection medium. A pair of electrodes 202 and 204 connected to a power supply 206 having positive and negative connectors 207 and 208, respectively, via conductors 209 and 210 and a switch 212 provide current for transport of glucose from a tissue surface into reservoir 200. The switch 212 may be controlled manually or by an automatic controller 213 (not shown). An analyzer 214 associated with the monitor measures the glucose concentration in glucose collection reservoir 200 and computes a glucose flux. A calibrator 216 correlates the computed glucose flux with blood glucose concentration.

Figures 1B-1D are drawings of a preferred embodiment of this invention. As shown in Figure 1B, two electrode/reservoir assemblies 110 are attached to collection sites 112 on a tissue surface 114 of a patient. The electrode/reservoir assemblies 110 are connected to a power supply 120.

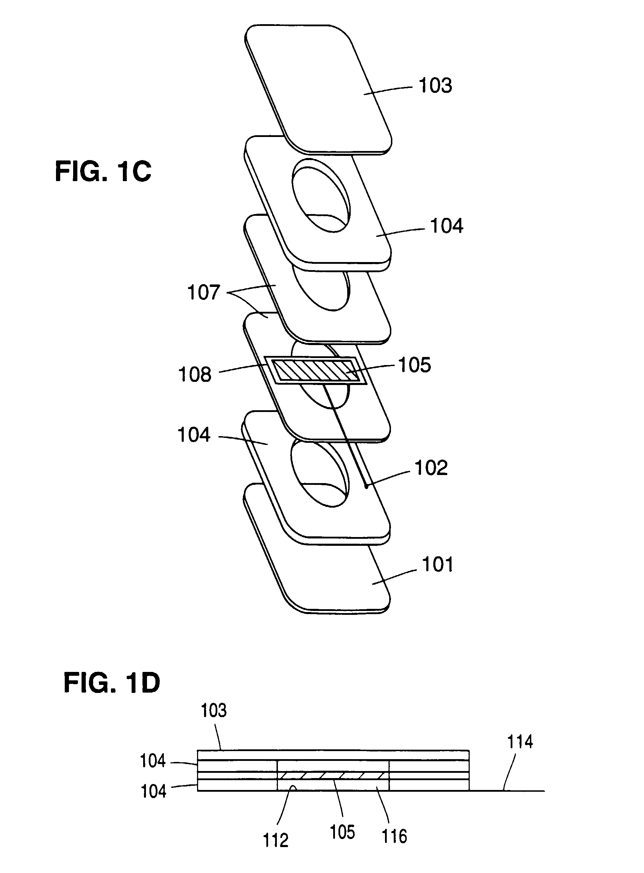

Figure 1C is an exploded view of the electrode/reservoir assembly 110. Element 101 is a release liner which is a protective layer for the adhesive of the skin contacting surface of the patch; element 102 is a sample port which can be a tube through which samples are removed; element 103 is the top layer, which can be a polymeric layer, which forms the upper housing for the collection reservoir/sample chamber and makes it possible to view the condition of the electrode as the treatment progresses; element 104 is a foam layer coated on one side with an adhesive which prevents contact of the electrode with the skin and serves as the walls of the collection reservoir/sample chamber; element 105 is an electrode which serves as either the cathode (*i.e.*, electron donor) or the anode (*i.e.*, electron receiver) depending on the polarity of the electrodes; element 107 is a transfer adhesive layer which holds the electrode 105 and the sample port 102 in place; and element 108 is a protective layer positioned between the electrode 105 and the skin to prevent the electrode from contacting the skin.

As illustrated, layers 104 and 107 each comprise part of the housing of the collection reservoir and allow for the collection reservoir to be open to the skin.

The release liner 101 is a protective layer for the adhesive of the skin contacting surface of the patch. This liner can be any suitable flexible material conventionally known in the art. It should be strong enough not to be torn away by normal movement but easily removed by the application of some manual effort.

The sample port 102 can be a tube through which samples are removed.

The top layer 103 can be any flexible material conventionally known in the art, such as a polymeric layer, which forms the upper wall of the housing of the collection reservoir/sample chamber and is preferably transparent, which makes it possible to view the condition of the electrode as the treatment progresses. Any transparent polymeric material conventionally used as the top layer in transdermal patches can be used. Preferably, the top layer is made of polyester or preferably polyethylene.

The foam layer 104 is any medical grade foam layer (foam tape) coated on one side with an adhesive which prevents contact of the electrode with the skin and serves as the walls of the sample chamber. Any coated foam layer (foam tape) which is conventionally used in transdermal patches which is compatible with the tissue and material to be sampled can be used, preferably the foam tape is a closed-cell polyolefin foam coated on one side with an acrylate adhesive, such as 3M # 9772.

The electrode 105 may be made of Ag/AgCl or any other electrode material. It is attached to the power source via electrical wires/leads.

The transfer adhesive layer 107 is a transfer adhesive layer which holds the electrode and the sample port in place. The adhesive can be any adhesive conventionally known in the art which can hold the layers together with the electrodes between. For example, the adhesive can be 3M # 1524, Medical Transfer Adhesive.

The protective layer 108 is positioned between the electrode 105 and the skin to prevent the electrode from contacting the skin. The protective layer is a polyurethane film coated on one side with an adhesive layer which keeps it in contact with the electrodes. Any conventional adhesive which is compatible with the skin can be used. For example, the protective layer is an Acutek tape (Flexcon™-Deraflex NRU 100 clear H566 spec 50K-9 (nylon reinforced cast polyurethane coated with an acrylic adhesive)).

Figure 1D is a cross-section of an electrode/reservoir assembly 110 in place at the collection site 112. A collection reservoir/sampling chamber 116 is preferably filled with an electrically conductive medium/fluid. Any fluid which is capable of conducting electric current between the electrodes can be used. Suitable fluids include water, aqueous saline solutions and preferably aqueous buffered saline solutions having a pH in the range of from about 4 to about 9. A preferred fluid is an aqueous sodium bicarbonate buffered sodium chloride solution having a pH of from about pH 7 to about pH 9. Obviously, the fluid is one that is inert to the material desired to be sampled (*i.e.,* glucose) and compatible with skin. The collection medium provides an electrical circuit connection between electrode 105 and the tissue surface.

Instead of the collection reservoir described above, a collection matrix may be provided, as in known in the art. Also, electrodes in direct contact with the skin may be used in place of the electrodes immersed in conductive fluid as described above.

Preferably, power supply 120 comprises a means for reversing the polarity of the power source during the sampling, such as a manual or automatic programmable switch. One suitable power supply is the Iomed Phoresor™ iontophoretic power supply.

Also, when monitoring is conducted for more than 15 minutes, the data is self-consistent, with data at the cathode usually being the most efficient when not using the alternating polarity embodiment. One of skill in the art can recognize that there is variation between subjects or even for a given subject and for use of different iontophoretic device designs, surface area, current density, and the like as to background glucose, time lag and drift. These can be determined by standard methods and the correlation of the data adjusted for background glucose, time lag and drift for an individual and/or device.

Another apparatus according to the present invention consists of one or more reservoirs which contact the skin or other epithelial layer. While the present invention is intended to be used on any epithelial layer, some surfaces may be more desirable depending upon the target substance to be removed. For example, mucosal membrane has a lower barrier for nonionized substances and also typically has a higher concentration of blood vessels, making it more a more desirable substrate for the removal of uncharged substances.

The reservoirs each contain an ionically conductive medium. The conductive medium is preferably a hydrogel which contains ionic substances in an amount sufficient to produce high ionic conductivity. The composition of a gel according to a preferred embodiment of this invention is described in detail below. In an alternative embodiment, the ionically conductive medium could be a wicking material such as sponge, gauze pad, or other material that is soaked with an ionically conductive electrolyte solution, such as a 0.5% NaCl solution buffered to pH 8.0 with 0.5% sodium bicarbonate.

A first iontophoresis electrode contacts one reservoir (which is in contact with the subject's epithelial membrane) and a second iontophoresis electrode contacts either a second reservoir in contact with the epithelial membrane or some other ionically conductive medium in contact with the epithelial membrane. A power source provides an electric potential between the two electrodes to perform reverse iontophoresis on the epithelial membrane in a manner known in the art. Preferably, a sensor selected to sense the presence, and possibly the level, of a target substance within a reservoir is in contact with the reservoir. A display communicating with the sensor provides an indication of the presence and (possibly) the level of the target substance within the reservoir. If suitably calibrated, the display can indicate concentration of the target substance within the subject's blood. This monitoring can be performed substantially continuously, periodically, or on demand.

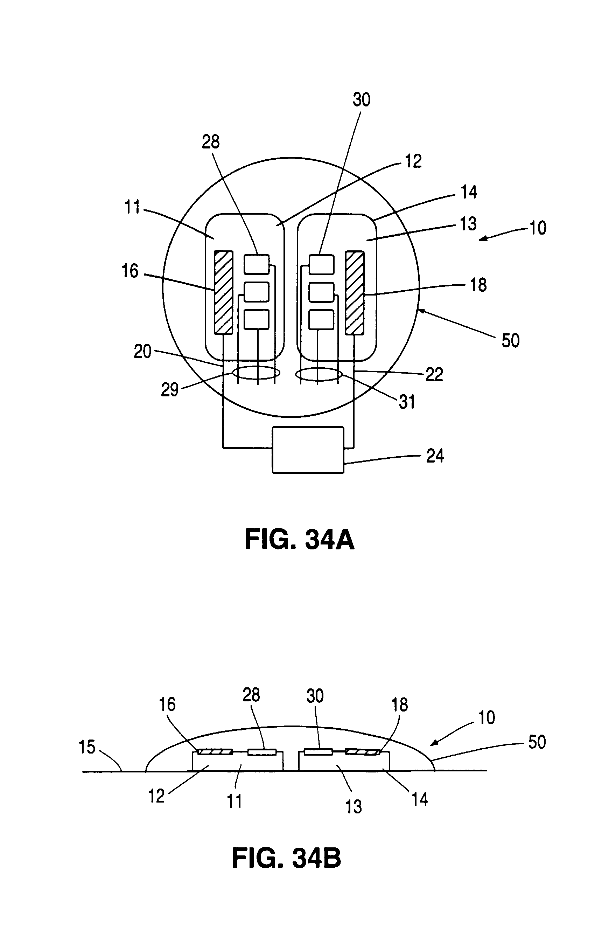

In Figure 34, two iontophoresis electrodes 16 and 18, preferably made of Ag or Ag/AgCl, are in contact with reservoirs 12 and 14. Each reservoir contains a conductive medium 11 and 13, each of which is in contact with the subject's epithelial membrane 15. In the preferred embodiment, the conductive medium of both reservoirs is a hydrogel. Alternatively, the conductive medium is an ionically conductive solution within a wicking material. A power source 24 provides an iontophoretic electric potential.

Also in contact with the conductive medium of both reservoirs are sensor electrode assemblies 28 and 30 to be used with a sensor apparatus (such as a potentiostat, not shown) to detect and/or quantify the extracted substances within the ionically conductive medium. Sensor electrode assemblies 28 and 30 may be connected to the sensor apparatus through conductors 29 and 31, respectively. Many types of electrode-based sensors and sensing methods are well-known in the art. For example, if the extracted substance of interest is glucose, a glucose sensor could be made by incorporating an enzyme specific to glucose in the ionically conductive medium and placing two or three electrodes in contact with the ionically conductive medium to detect the product of the reaction between glucose and the enzyme specific to glucose.

A housing 50 preferably surrounds both reservoirs and both sets of electrodes. Housing 50 is designed to hold the conductive medium in contact with the skin. The housing may also provide access for the electrical means and the sensor.

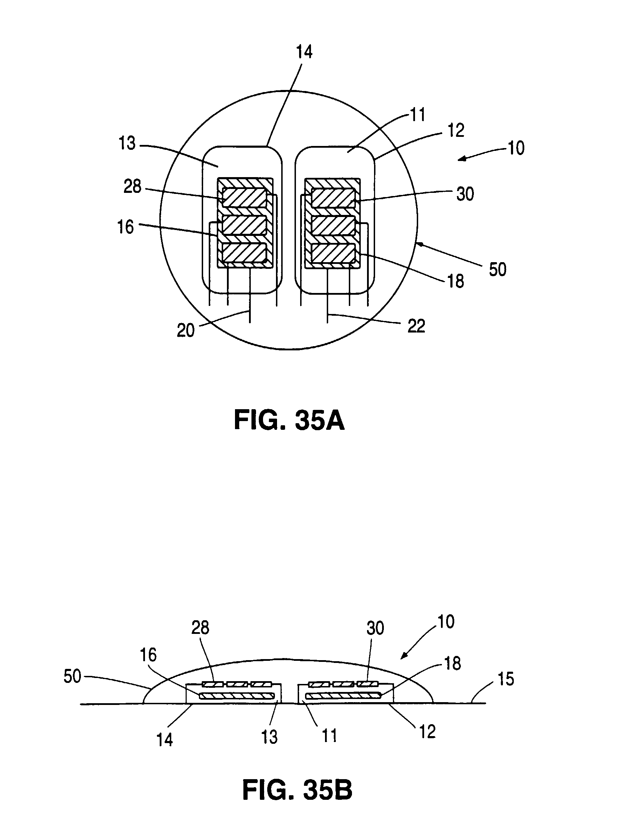

An alternative embodiment is shown in Figure 35. In this embodiment, the sensor electrode assemblies 28, 30 are disposed on the conductive medium of the reservoirs above the iontophoresis electrodes 16 and 18. The preferred conductive medium is once again a hydrogel, although the combination of a wicking material and an ionically conductive medium may be used instead. In this embodiment, the iontophoresis electrodes are porous or made of a mesh-like material. The extracted substance can diffuse through the iontophoresis electrodes to the sensor electrode assemblies 28 and 30. The porous electrodes can be made of, for example, plastic, ceramic, metal mesh, or combinations thereof.

The pores of the electrodes should range in size from about 200 µm to 1 mm. Optimally, the pores should be > 500 µm in size. Ideally, the number of pores is sufficient to give the electrodes a high porosity. Additionally, the surface of the pores may be hydrophilic. The hydrogel is drawn into the porous electrode via capillary action, thus creating ionic conductivity between the electrodes.

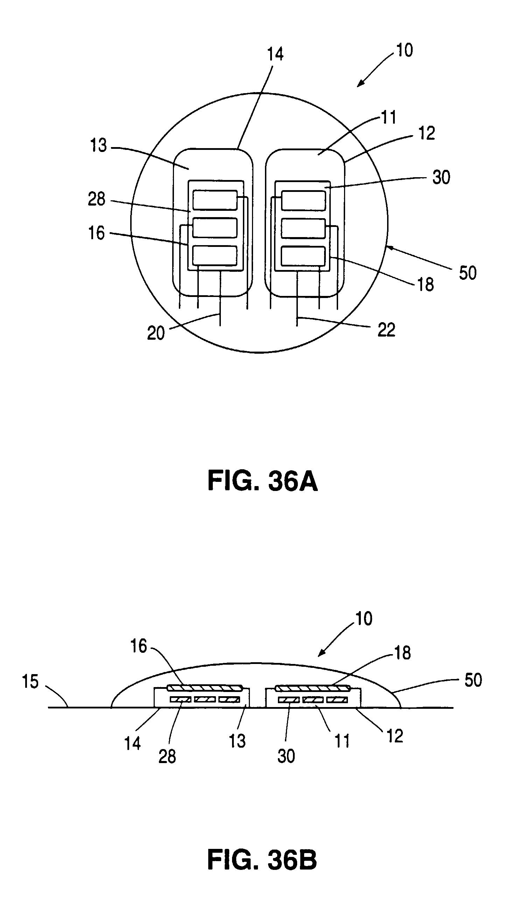

In an alternative embodiment shown in Figure 36, the sets of sensor electrode assemblies 28 and 30 are disposed within each reservoir's conductive medium beneath the iontophoresis electrodes 16 and 18. The sensor electrodes are therefore preferably porous (made, e.g., of a mesh-like material), as described above with respect to the iontophoresis electrodes shown in Figure 35. Once again, while the preferred conductive medium is a hydrogel, an ionically conductive medium in a wicking material may also be used.

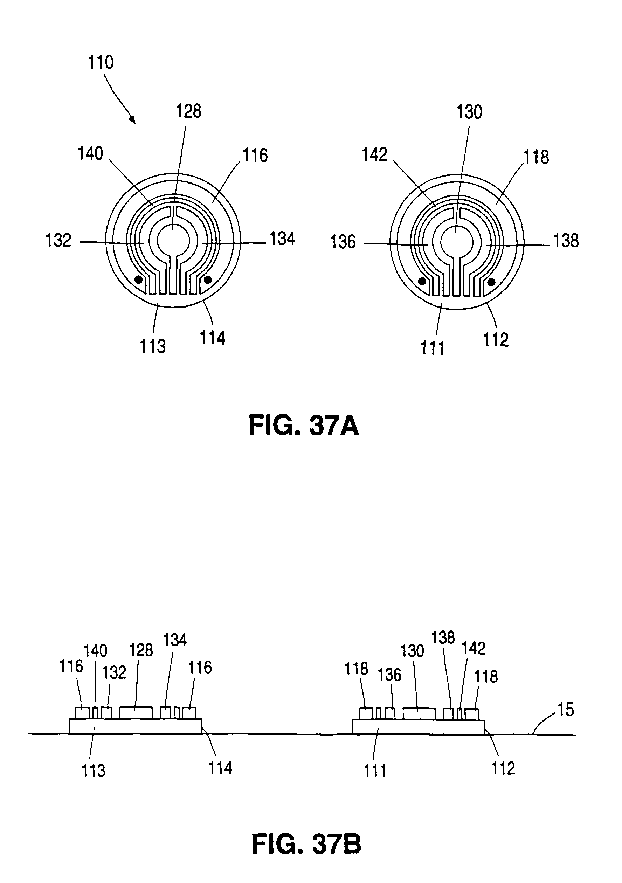

In yet another alternative embodiment, the electrodes are arranged as shown in Figures 37A and 37B. Figures 37A and 37B show an iontophoretic collection apparatus 110 with two reservoirs 112 and 114 each having a conductive medium 111 and 113, preferably cylindrical hydrogel pads. Mounted on conductive media 111 and 113 are ring-shaped iontophoresis electrodes 116 and 118. Electrode 116 surrounds three sensor electrodes 128, 132 and 134. The sensor working electrode 128 is surrounded by the reference electrode 132 and counter electrode 134, which are arc-shaped. A guard ring 140 runs between the sensor electrodes and the iontophoresis electrode 116 to minimize signal noise from the iontophoresis circuit. Conductors (not shown) run from the electrodes and the guard ring to outside power and ground sources and a sensor apparatus (not shown). Reservoir 114 has a similar electrode arrangement. This embodiment has the advantage of using most of the available space in the hydrogel pad while maximizing the size of the sensor working electrode, which in turn will maximize the signal from the sensor. As in the other embodiments, a wicking material soaked with an ionically conductive medium may be used in place of the hydrogel pads.

While Figure 37 shows cylindrical collection reservoirs and ring-shaped and arc-shaped electrodes, it should be understood that other reservoir and electrode geometries may be used as well without departing from the scope of this invention.

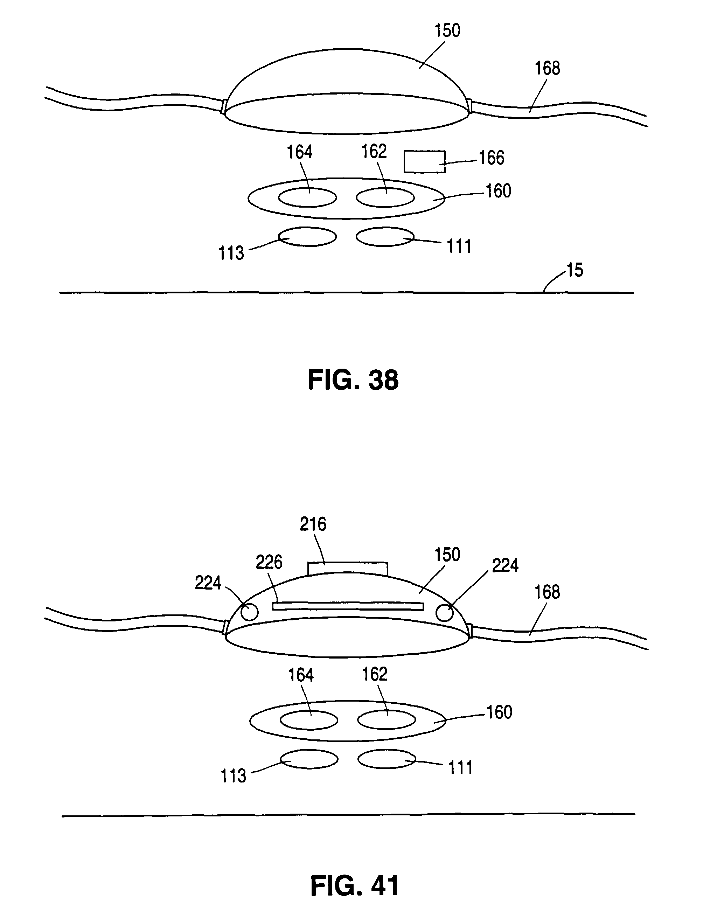

A suitable housing for the embodiment of Figure 37 is shown in exploded form in Figure 38. When assembled, the overall size and appearance of this embodiment is similar to a wristwatch.

The electrode assemblies of Figure 37 are represented in Figure 38 as elements 162 and 164 on an electrode board 160. In other words, electrode assembly 162 of Figure 38 corresponds to electrodes 118, 130, 136, 138 and 142 of Figure 37, and electrode assembly 164 of Figure 38 corresponds to electrodes 116, 128, 132, 134 and 140 of Figure 37. A connector 166 provides the electrical connection between the electrodes and the power source, ground source, and sensor apparatus.

In this embodiment, the reservoirs 112 and 114 are separable from their respective electrode assemblies 162 and 164. When assembled, however, the conductive media are in contact with the electrode assemblies. Electrode board 160 and conductive media 111 and 113 are disposed within housing 150. In use, straps 168 hold the apparatus against the subject's skin 15 so that pads 111 and 113 are in contact with the skin. In addition, the power source (e.g., rechargeable or nonrechargeable batteries) may be disposed on the strap. In the preferred embodiment, the conductive media 111 and 113 are hydrogel pads, although the wicking material/ionically conductive medium combination may also be used.

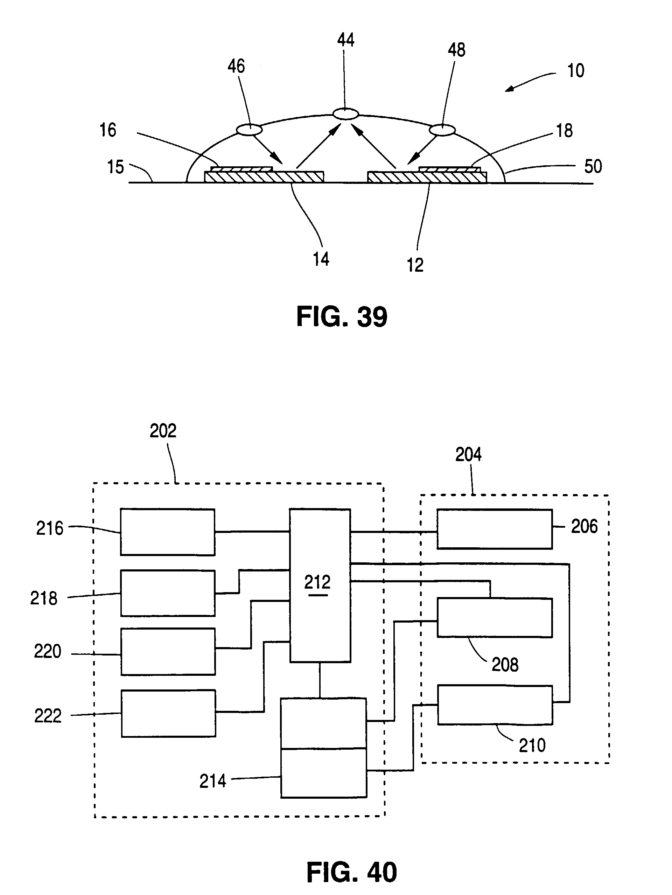

In yet another alternative embodiment, an optical sensor can be employed as shown in Figure 39. The optical sensor need not be in direct contact with the ionically conductive medium. Alternatively, two sensors can be incorporated, one physically close to each iontophoresis electrode to measure the substance extracted at each electrode. These sensors may be selective for the same extracted substance or different extracted substances. The embodiment depicted in Figure 39 utilizes an optical colorimetric sensor 44 to detect and measure the extracted substance. The intensity of light generated by the light source 46, 48 is reflected from the conductive media (e.g., hydrogel pads) containing the color change chemistry. The light source 46, 48 are LEDs. Additionally, a baffle (not shown) may be placed between the light source and the detector to prevent light from reaching the detector directly. TiO₂ (or other reflective material) can be added to the conductive media to increase the baseline reflectance.

In this embodiment, the inside of the housing 50 may be colored flat black to reduce the stray light reflections that may occur when the sensor employed is an optical sensor. Alternatively, the housing may be shaped and mirrored in order to act as a light-directing element to direct the maximum amount of LED intensity onto the hydrogel and to collect the maximum amount of reflected light onto the detector for the Figure 39 embodiment discussed below.

In an embodiment where the conductive medium is a relatively transparent and translucent hydrogel, a double pass absorbance cell (not shown) may be created by placing a reflective material, such as TiO₂ coated porous membrane or Al₂O₃ porous membrane, on the skin side of the hydrogel. In this embodiment, the light from the LED would pass through the hydrogel, reflect, and pass through the hydrogel again.

In any of the hydrogel or wicking material embodiments, the conductive medium is preferably attached to the epithelial membrane using an adhesive. The adhesive properties can be inherent in the conductive medium (as is the case for some hydrogels) or added to the conductive medium through a separate adhesive element. If the conductive medium is not sufficiently adhesive, a separate means of holding the conductive medium in contact with the skin (such as a strap) may be used.

In the embodiments described above, the reservoirs are physically separated within housing 50. If necessary, a non-porous, electrically insulating element can be placed between the two ionically conductive media to prevent contact.

The hydrogel used in this invention must (1) be ionically conductive, (2) provide a hospitable environment for the sensor enzyme, and (3) not interfere with sensor operation. An example of the preparation of a suitable ionically conductive and biosensing hydrogel for use in monitoring glucose with the embodiments discussed above is as follows. NaCl and NaHCO₃ (food grade) are dissolved in water to make a solution in the range of about 0.1% to about 1.0%, preferably about 0.5%. Cross-linked acrylic acid polymer (preferably, Carbopol 934 PNF, available from B.F. Goodrich) is suspended into the NaCl·NaHCO₃·H₂O solution. The pH of the resulting solution is tested with an Oaktron pH Tester 1, and then adjusted to from about pH 4.0 to about pH 10.0, preferably from about pH 6.0 to about pH 8.0, using food grade sodium hydroxide (available from Malinckrodt). The actual pH desired depends on the enzyme to be used for the biosensor; the ranges provided here are for glucose detection. Glucose oxidase in a range from about 50 I.U. to about 100,000 I.U., preferably from about 300 I.U. to about 5000 I.U., is added to the pH-adjusted NaCl·H₂O·Carbopol solution. The actual amount of glucose oxidase depends on the desired shelf life of the product, the intended use life of the product, and the stability of the enzyme during storage.

Additional substances may be added to the solution. These include, but are not limited to, for example, other salts, other buffers, tackifiers, biocides, preservatives and enzyme stabilizers.

In an alternative embodiment, it is possible to store the conductive media in a dry form. Dry forms include, but are not limited to, a dehydrated gel, or a sponge containing the components necessary to allow proper operation of the device. Water, or electrolyte solution could then be added to the medium before the conductive media are attached to the skin, thus rehydrating the medium. An advantage of dry storage is that it allows for greater stability of the components of the medium during storage and also allows for longer shelf life.

In practice, an electric potential (either direct current or a more complex waveform) is applied between the two iontophoresis electrodes 16 and 18 such that current flows from the first electrode through the first conductive medium into the skin, and back out from the skin through the second conductive medium to the second electrode. This current flow extracts substances through the skin into one or both reservoirs 12 and/or 14 through the process of reverse iontophoresis or electroosmosis. The electric potential may be applied as described in the priority application of the present application, U.S. Patent Application S.N. 08/265,048, "Method of Continuous Iontophoretic Measurement of Chemical Species in Body Fluids," filed June 24, 1994, now abandoned.

As an example, to extract glucose, the applied electrical current density is preferably in the range of about 0.01 to about 2 mA/cm². In a preferred embodiment, in order to facilitate the extraction of glucose, electrical energy is applied to the electrodes 16, 18, and the polarity of the electrodes is alternated at a rate in the range of about 0.1 Hz (one switch every 10 seconds) to about 1 switch every hour, so that each electrode is alternately a cathode or an anode.

The polarity switching can be manual or automatic. The polarity switching can be of any frequency, especially at frequencies between about 1 cycle per 20 seconds to about 1 cycle pe-r 4 hours, preferably between about 1 cycle per 20 seconds to about 1 cycle per 2 hours, more preferably between about 1 cycle per minute to about 1 cycle per 2 hours, or between about 1 cycle per 10 minutes to about 1 cycle per hour, and especially about 1 cycle per half hour. By cycle is meant one interval of current in each direction. The application of electrical energy in a given cycle may cease during glucose analysis of the collection medium.

As discussed above, the iontophoresis electrodes are connected to a power source and the sensor electrodes are connected to a sensor apparatus. Figure 40 is a circuit block diagram showing the elements of an iontophoretic monitoring system according to a preferred embodiment. Incorporated into the collection reservoir portion 204 of the system are the iontophoresis electrodes 208 and the sensor electrodes 210. These electrodes are in contact with the conductive medium of each reservoir. Also included is an optional temperature sensing element 206 (such as a thermistor) which monitors the temperature at the collection reservoir to enable temperature correction of the sensor signal, if necessary.

The power and control portion 202 of the system includes a controller 212 (such as a microprocessor), a power supply 214, and a display (or other output) 216. The display may be used, for example, to allow patients to scroll through their present and previous analyte (e.g., glucose) level readings and to alert patients to fluctuations in their levels. The power supply 214 is shown as having two parts, one for the iontophoresis function and one for the sensing function. The two parts may not be necessary, of course, depending on the approach chosen for the iontophoresis and sensing functions. Also included in the system are an optional alarm 218, optional input/output ports 220 and a memory 222 to store and recall data, such as, e.g., periodic glucose readings.

In one embodiment, the collection reservoir portion 204 of the system is contained in one housing (such as the housing 150 shown in Figure 38), and the power and control portion 202 of the system is contained in a separate housing. Electrical conductors connect the two housings to carry power, control signals and data.

An alternative embodiment is shown in Figure 41. In this embodiment, the power and control elements are contained within the same housing as the collection reservoir portion of the system. A display 216 is provided at the top of the housing 150. Batteries 224 are part of the power supply. The microprocessor, memory and other circuit components are mounted on PC board 226 disposed within housing 150.

A process of using the iontophoretic collection system of this invention to monitor blood glucose concentration is as follows. This example uses two hydrogel pads made substantially as described above for the conductive media of the collection reservoirs, the electrode assembly shown in Figure 37 and the housing arrangement shown in Figures 38 and 41.

The monitoring process begins by placing two hydrogel pads in contact with the electrode assembly within the housing. The housing is then strapped to the subject so that the pads are in contact with the subject's skin. Power is then provided to the iontophoresis electrodes to begin the collection process.

As glucose travels across the subject's skin into the hydrogel pads, the glucose reacts with the glucose oxidase within the pads to generate hydrogen peroxide. The presence of hydrogen peroxide generates a current at the sensor working electrode that is proportional to the concentration of the hydrogen peroxide within the pad. The current provides a signal that is interpreted by the system controller to provide a glucose concentration value for display. This current may be correlated with the subject's blood glucose concentration so that the system displays the subject's actual blood glucose concentration as measured by the iontophoretic collection system. For example, the system can be calibrated to the subject's actual blood glucose concentration by sampling the subject's blood through the standard pin prick technique, analyzing the blood glucose (using, e.g., a blood glucose monitor such as One Touch™ Basic, LifeScan, Milpitas, CA), and correlating the glucose concentration measured in the blood with the iontophoretically extracted glucose concentration in the reservoir. The monitoring can be performed substantially continuously, periodically, or on demand.

The hydrogel pads contain an amount of glucose oxidase sufficient to provide a predetermined monitoring period. After that period expires, the user should replace the used hydrogel pads for new ones. This invention facilitates that operation by providing a design in which the hydrogel pads are easily separable from the rest of the iontophoretic collection system.

In an alternative embodiment, the system can be provided with a system that determines whether a hydrogel pad is operational or not. For example, a new pad contains virtually no hydrogen peroxide (e.g., below 80 µM) prior to activation of the iontophoretic collection process. If the system detects the presence of hydrogen peroxide above a threshold amount upon start-up (i.e., before the application of any iontophoretic potential to the iontophoresis electrodes), it can warn the user (e.g., by activating an alarm) that the pad should be replaced with a new pad.

In another alternative embodiment, the two hydrogel pads can be formed as a single element, such as by incorporating an insulator to separate the hydrogel into two functional elements.

Other modifications will be apparent to those skilled in the art.

### EXAMPLES

The following examples are provided to illustrate the invention but should not be regarded as limiting it in any way.

### Example 1

A continuous iontophoretic extraction was conducted on subjects who fasted 12 hours overnight prior to the start of the experiment and during the experiment. In these experiments, two adhesive sampling patches, each with Ag or AgCl electrodes inserted into the aqueous sampling chamber of the patches were applied to the forearm. The iontophoretic patches had a collection surface area of 2.85 cm² per patch and a collection reservoir volume of 0.4 mL per patch. The chambers were filled with 0.45% sodium chloride in water, U.S.P. For the direct current or DC protocol (the standard protocol), an electric current of density 0.32 mA/cm² was applied for 15 min, followed by a 5 min rest interval in which the saline was removed for analysis and then replaced with fresh saline. During the 5 min rest period, blood glucose was measured by a standard finger prick test (One Touch Basic, LifeScan, Milpitas, CA) method for comparison to iontophoretically extracted glucose. The 15 min iontophoresis and 5 min rest/sampling procedure was repeated for 3 to 5 h. The iontophoretically extracted samples were analyzed by HPLC.

For the passive controls, the same procedures were used except that no current was applied.

The results of these experiments are set forth in Figures 2 and 3. In each figure, blood glucose concentration in mg/dL is given versus time in minutes. The open square is for blood glucose, solid triangle is for iontophoretic glucose at the anode, the solid square is for iontophoretic glucose at the cathode and the open circle for passive glucose.

The data in Figures 2-3 demonstrated that (1) continuous extraction washed out interference from glucose in the skin (stratum corneum, epidermis, or dermis), (2) the steady state was reached in less than one hour, (3) there was a drift in glucose flux, especially at the cathode and (4) cathode flux was greater than anode flux which was greater than passive flux.

### Example 2

An oral glucose tolerance test was conducted on non-diabetic subject (1) who had fasted prior to the experiment for 12 h overnight. One hour into the test, 75 gm of glucose was administered orally. Blood glucose levels increased from 100 to 180 mg/dL. Iontophoresis was administered and glucose was monitored as described in Example 1, using the standard DC protocol. The results of the test is set forth in Figures 4-10.

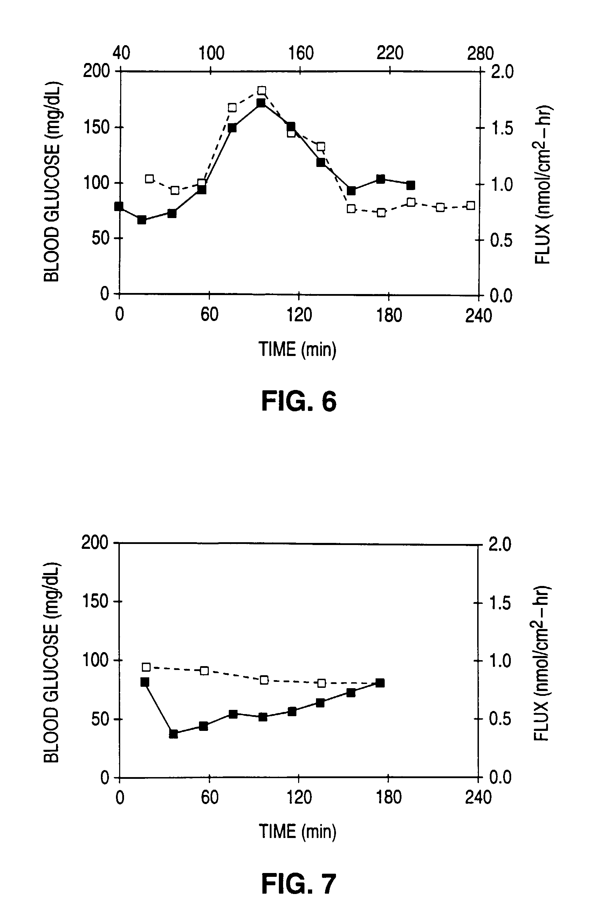

Figure 4 shows the results of the experiment. In each figure, blood glucose concentration in mg/dL is given versus time in minutes. The open square is for blood glucose, solid triangle is for iontophoretic glucose at the anode, the solid square is for iontophoretic glucose at the cathode and the open circle for passive glucose. Figures 5 shows the same data as in Figure 4, but with only data for the cathode (the best response) shown. Figure 6 shows the same data as in Figure 5, but with the iontophoretically extracted glucose "shifted" 40 minutes compared to the blood glucose, illustrating a 40 minute time lag. In Figure 6, it is seen that the extracted glucose flux was not completely correlated to blood glucose, even with the time lag. The flux was relatively lower compared to the blood glucose at the start of the experiment and relatively higher than the blood glucose at the end of the experiment. Figure 7 shows that same data as Figure 2, but with only the data for the cathode shown. Figure 8 shows a linear fit to the data in Figure 7. The data in Figure 8 excluded the extraction samples obtained before one hour into the extraction to eliminate the interference from glucose in the skin prior to the start of the experiment. Figure 9 shows the same data as Figure 5, but with the equation for the linear fit of Figure 8 subtracted from the data of Figure 5 to adjust for drift of glucose flux values over time. Figure 10 shows the data of Figure 6, adjusted for drift. Figure 10 demonstrates that these was a strong correlation between lag adjusted and drift adjusted iontophoretically extracted glucose and blood glucose.

Overall, the data in Figures 4-10 demonstrated that (a) there was correlation between glucose extraction at the cathode and blood glucose, (b) cathode flux was greater than anode flux which was greater than passive flux, (c) there was a time lag of about 40 min between changes in blood glucose and changes in extracted glucose, and (d) there was linear drift that was independent of blood glucose.

### Example 3

Oral glucose tolerance tests were conducted on subjects (3), (4), and (5) following procedures similar to those described in Example 2 above. Results of these tests are set forth in Figures 11-16. In each Figure, blood glucose concentration in mg/dL is given versus time in minutes. The open square is for blood glucose, solid triangle is for iontophoretic glucose at the anode, and the solid square is for iontophoretic glucose at the cathode.

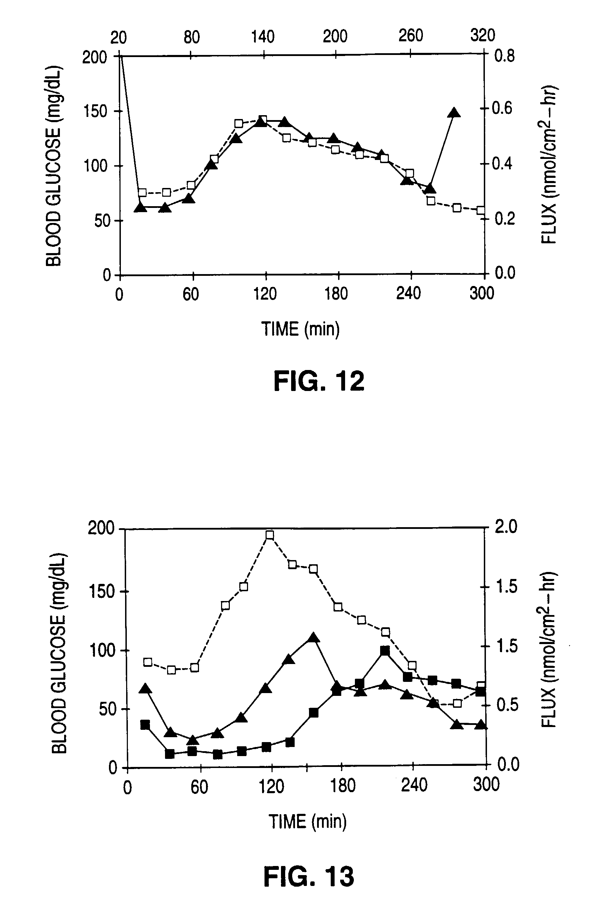

Figures 11 and 12 are for subject (3). The data demonstrates that (a) there was a slight correlation between blood glucose and iontophoretically extracted glucose at the cathode, but there was much better correlation at the anode, (b) cathode flux was greater than anode flux, (c) anode flux tracked blood glucose with a slight drift and a time lag of 20 minutes.

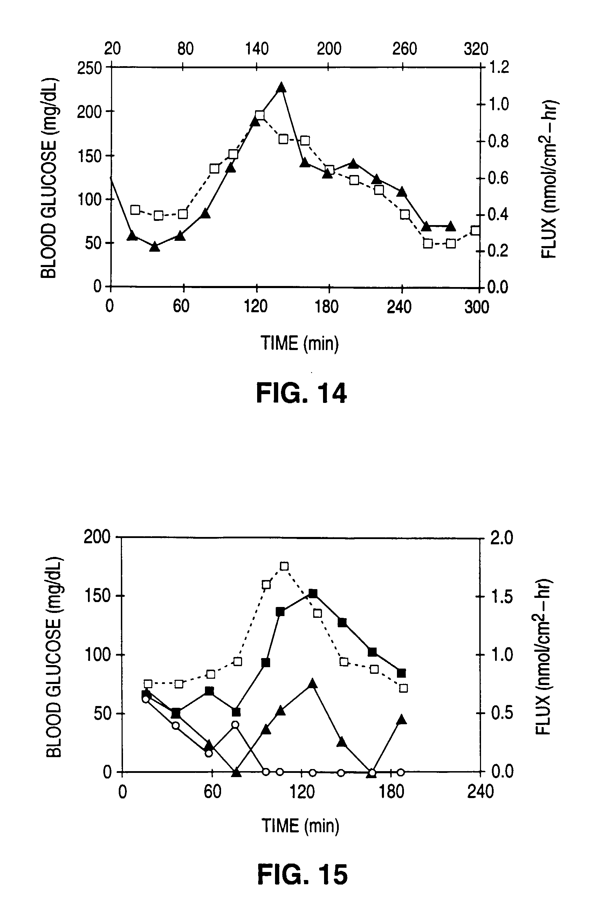

Figures 13 and 14 are for subject (4). The data demonstrates that (a) there was a slight correlation between blood glucose and iontophoretically extracted glucose at the cathode, but there was much better correlation at the anode, (b) unlike the other subjects, anode flux was greater than cathode flux, and (c) anode flux tracked blood glucose with a slight drift and a time lag of 20 minutes.

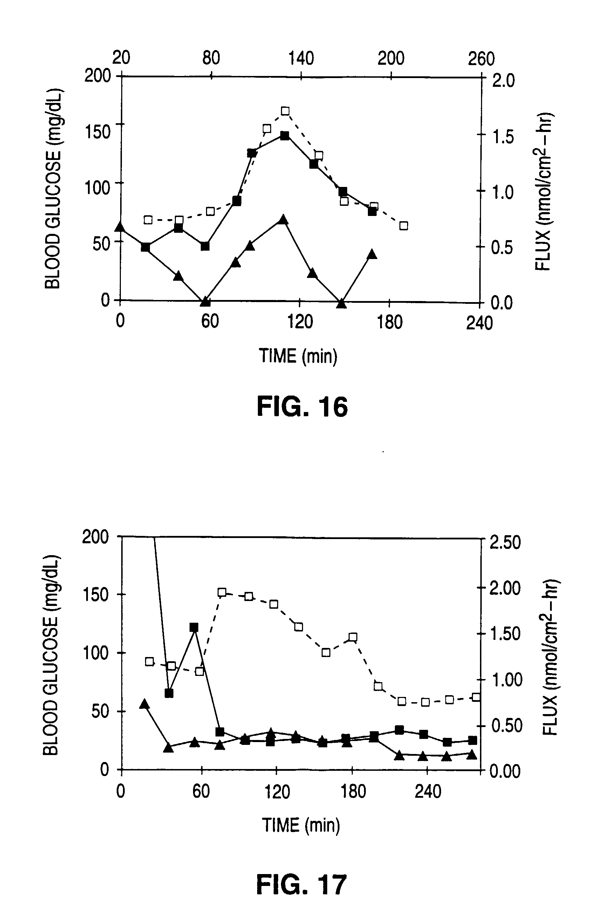

Figures 15 and 16 are for subject (5). The data demonstrates that (a) there was a correlation between blood glucose and iontophoretically extracted glucose at both the anode and the cathode, (b) cathode flux was greater than anode flux, (c) both cathode and anode fluxes tracked blood glucose with a slight drift and a time lag of 20 minutes.

### Example 4

Blood glucose monitoring experiments were performed in which a comparison was made between a standard iontophoretic protocol consisting of direct current between an anode site and a cathode site versus an alternating polarity iontophoretic protocol of the invention in which the electrodes act as both the anode and the cathode during the course of the extraction.

The alternating polarity protocol was identical to the standard protocol, except that the polarity of the power supply was switched during each 5 min rest period. In this way, the cathode chamber of one 15 min period became the anode chamber of the next 15 min period, and vice versa.

Oral glucose tolerance tests were conducted on subjects (6) and (7), following procedures similar to those described in Examples 2 and 3 above. Results of these experiments are shown in Figures 17-20. These figures are graphs comparing the results of the standard protocol and the alternating polarity protocol performed simultaneously on the same subject.

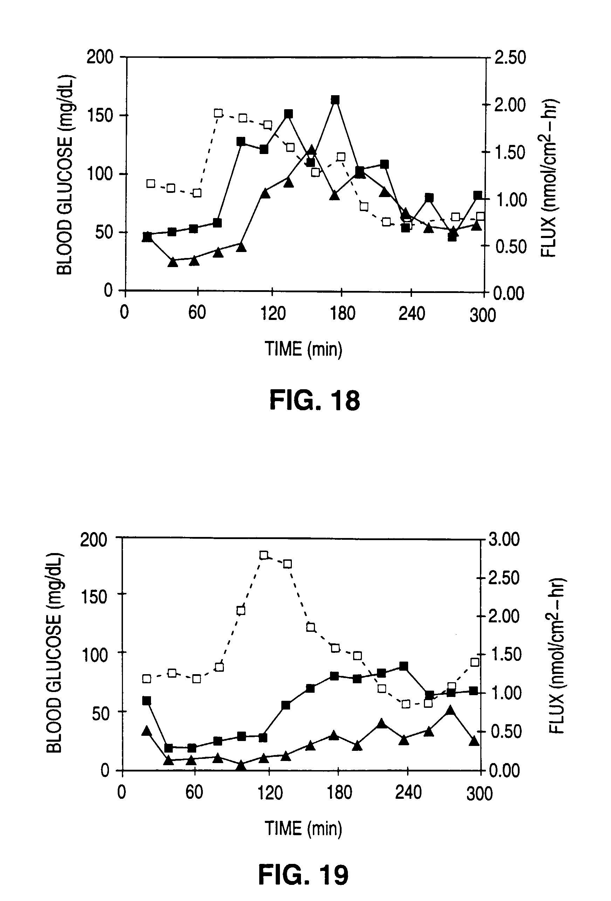

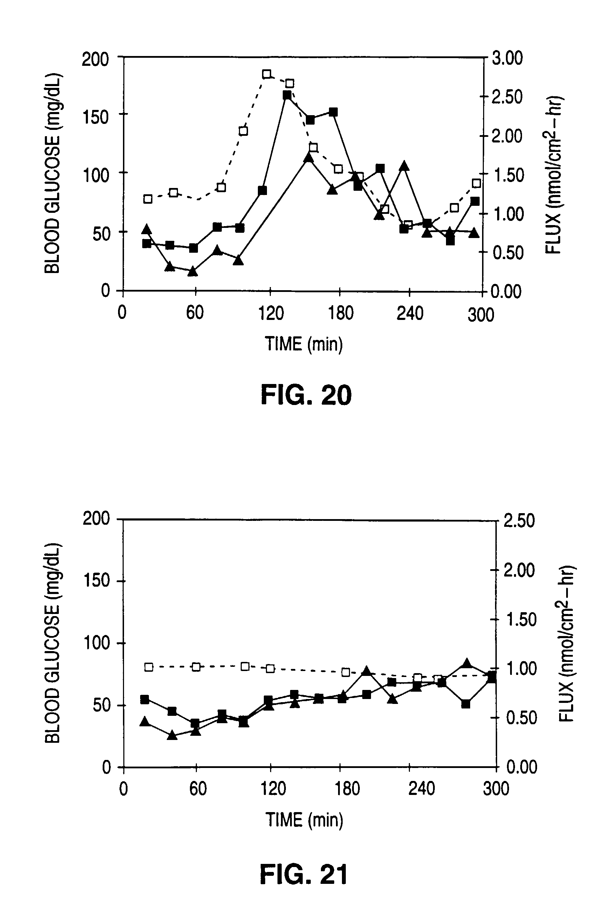

Figures 17 and 19 show the standard (DC) method which was performed on the right arm of subjects (6) and (7), respectively, and Figures 18 and 20 show alternating polarity protocol method, which was performed on the left arm of subjects (6) and (7), respectively. In each figures, blood glucose concentration in mg/mL is given versus time in minutes. The open square is for blood glucose, the solid triangle is for iontophoretic glucose at the anode, and the solid square is for iontophoretic glucose at the cathode.

As can be seen by comparing the Figures 17 and 19 with Figures 18 to 20, respectively, the alternating polarity method gave more response and better correlation, particularly for the anode response.

The above results demonstrate the improvement in sampling profiles from alternating polarity, or the use of a single sampling electrode that acts, alternatively, as anode and cathode or even both during a cycling period during the course of the extraction, as compared to the direct current standard (DC) mode of iontophoresis.

The following are results of tests in which the data is correlated for time lag and/or drift as discussed below.

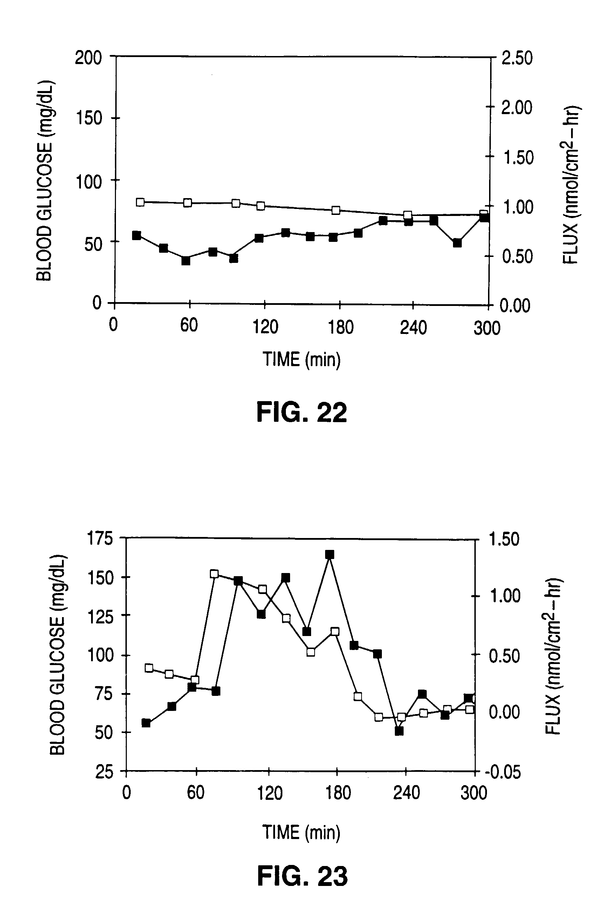

A continuous iontophoretic extraction was conducted on subject (6) as in Figure 18, but in a protocol in which the subject had fasted 12 hours overnight prior to the start of the experiment and during the experiment (no oral glucose tolerance test). The results are shown in Figures 21 and 22. Figures 21 and 22 demonstrate that there was a drift in the anode and cathode fluxes for subject (6).

Figure 23 is a graph of the results of a blood glucose monitoring of the same subject (6) as Figure 18, but with the flux values for the cathode in Figure 22 subtracted from the flux values for the cathode of Figure 18.

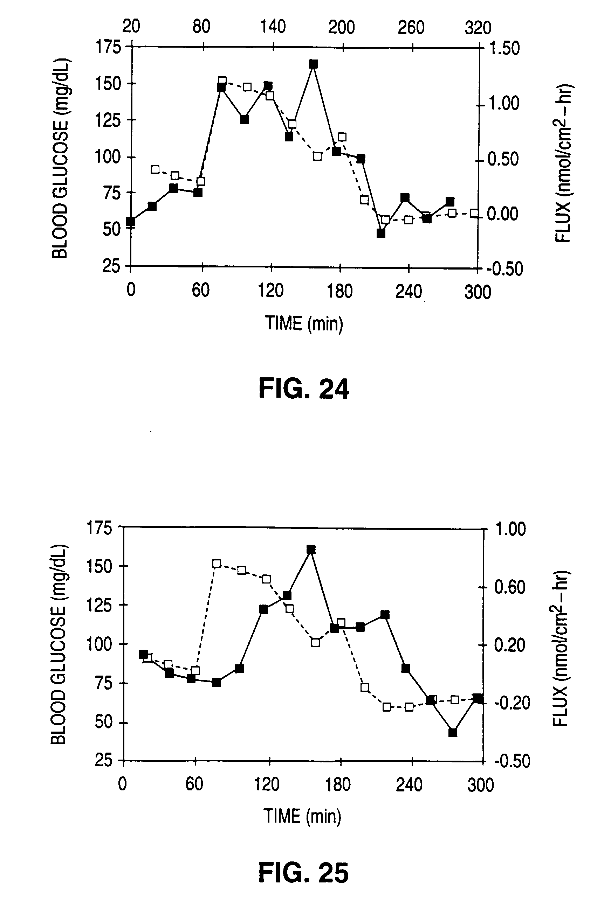

Figure 24 is a graph of the results of a blood glucose monitoring of the same subject (6) as Figure 23, but with the cathode flux adjusted for both a 20 minute time lag and the drift, as in Figure 22.

Figure 25 is a graph of the results of a blood glucose monitoring of the same subject (6) as Figure 18, but with the flux values for the anode in Figure 21 subtracted from the flux values for the anode in Figure 18.

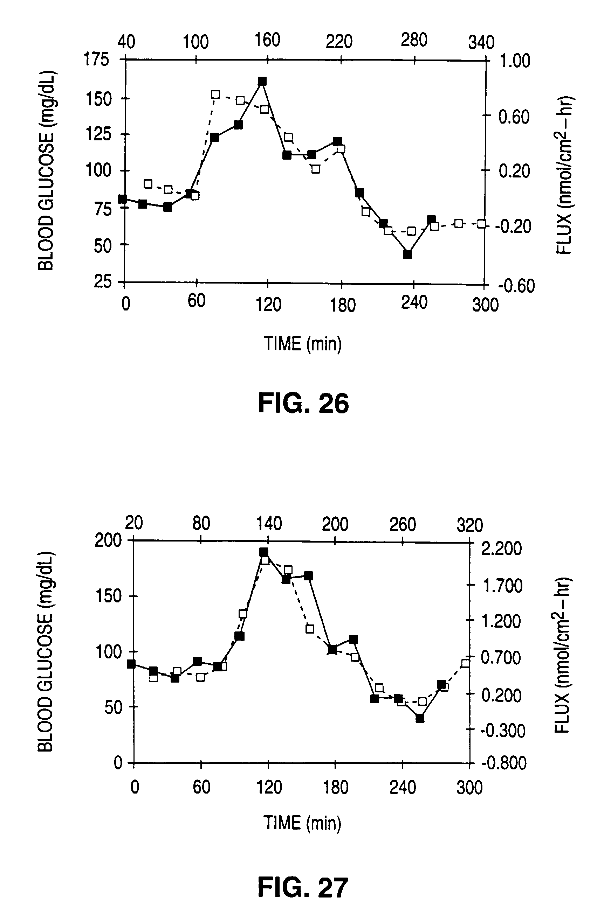

Figure 26 is a graph of the results of a blood glucose monitoring of the same subject (6) as Figure 25, but with the anode flux adjusted for both a 40 minute time lag and the drift, as in Figure 25.

Figure 27 is a graph of the results of a blood glucose monitoring of the same subject (7) as Figure 20 under the alternating polarity protocol, but with the cathode flux adjusted for time lag and a linear drift.

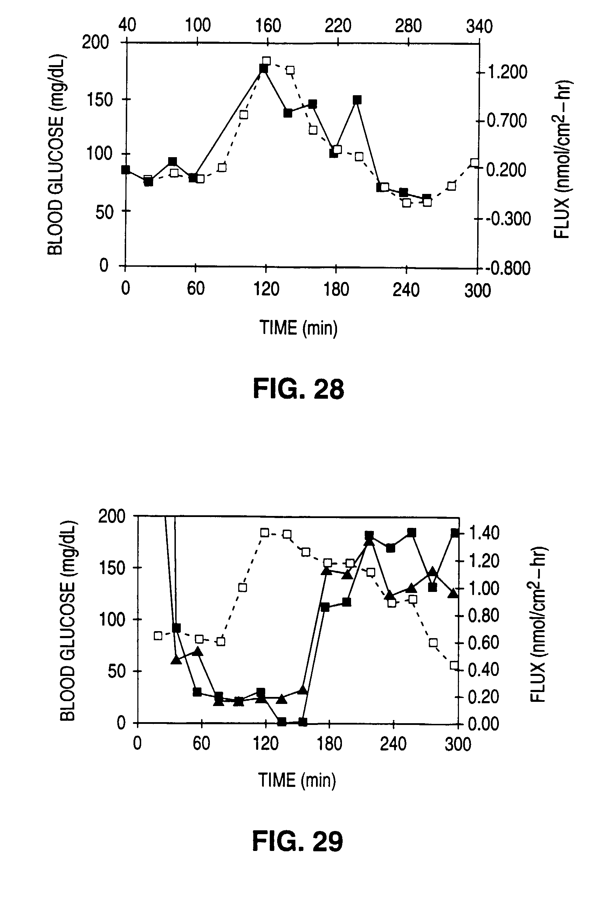

Figure 28 is a graph of the results of a blood glucose monitoring of the same subject (7) as Figure 20 under the alternating polarity protocol, but with the anode flux adjusted for time lag and a linear drift.

In total 12 normal subjects were tested. The results are summarized in Table 1 below.

**Table 1**

| | Direct Current Cathode Anode | | Alternating Polarity Cathode Anode | |
|---|---|---|---|---|
| Good tracking | 0 | 5* | 7 | 4 |
| Oscillation | 0 | 0 | 5 | 6 |
| Long Lag (>60 min) | 5 | 1 | 0 | 2 |
| No response | 7 | 6 | 0 | 0 |
| TOTAL | 12 | 12 | 12 | 12 |
| Good = tracking with drift = r² ≥ 0.5, lag ≤ 40 min | | | | |
| Long lag = r² ≥ 0.5, lag ≥ 60 min | | | | |
| Oscillation = qualitative tracking with oscillation (lag ≤ 40 min) | | | | |
| No response = r² ≤ 0.5 | | | | |

| | | | | |
|---|---|---|---|---|
| *Anode tracking, but low magnitude of flux | | | | |

The data demonstrates that there was a lower incidence of non-response and long time lag with the alternating polarity embodiment of iontophoresis administration.

### Example 5

Saline was injected into each of two adhesive sampling patches, each containing an electrode. A current of 0.32 mA/cm² was applied through the electrodes for 15 minutes. The saline was then removed, a blood glucose finger prick measurement was made, and fresh saline was reinjected during a 5 minute rest period. The procedure was repeated 15 times for a total of 5 hours. In this example, JK 1-17-94, the current was applied DC for 8 sample periods. On the 9th sample period, the current was switched to an alternating polarity protocol of a switch every 15 minutes. The results are set forth in Figure 29. There is a sharp and dramatic increase in flux as the protocol was switched from direct current to alternating polarity.

### Example 6

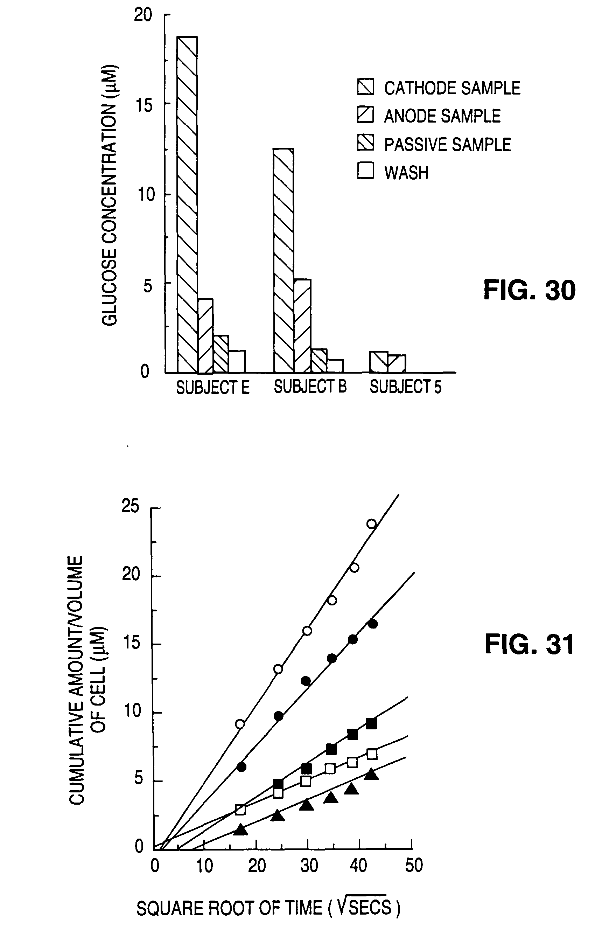

Glucose monitoring was conducted on the ventral forearm of subjects. The collection reservoir was a thin, flexible, plastic device of area 10 cm², holding a volume of 0.55 mL. The relatively large surface area-to-volume ratio provided reasonable extraction efficiency. The collection reservoir held 0.1 M sodium chloride, and housed Ag/AgCl electrodes. Iontophoretic current was delivered to the electrodes from a custom built, computer-controlled power supply. Sampling was conducted at 0.25 mA/cm² to avoid unnecessary sensation when the power supply was activated the current was "ramped" to its final constant value over a period of about 30 sec. Current was passed for periods of between 5 and 60 min. Prior to commencement of an experiment, the skin sites were left in contact with sodium chloride for 60 sec. This solution (the "wash") was then removed for analysis and replaced before activating current flow. Several control measurements (no-current) were also made. The most extensive of these involved repeated filling, emptying and replacement of the collection reservoir solutions as a function of time. This allowed determination of the passive extraction of cutaneous glucose. Analysis of glucose utilized a Dionex chromatographic system with pulsed amperometric detection. A 50 uL aliquot was used for each assay. The detection limit was 0.6 uM. Results of these experiments are set forth in Figures 30-31.

Figure 30 is the results of iontophoretic sampling of glucose at the cathode and anode compared to passive and wash controls.

Figure 31 is a graph of the cumulative amount of glucose extracted passively form the skin plotted as a function of the square-root of time. Each line corresponds to a single subject.

### Example 7

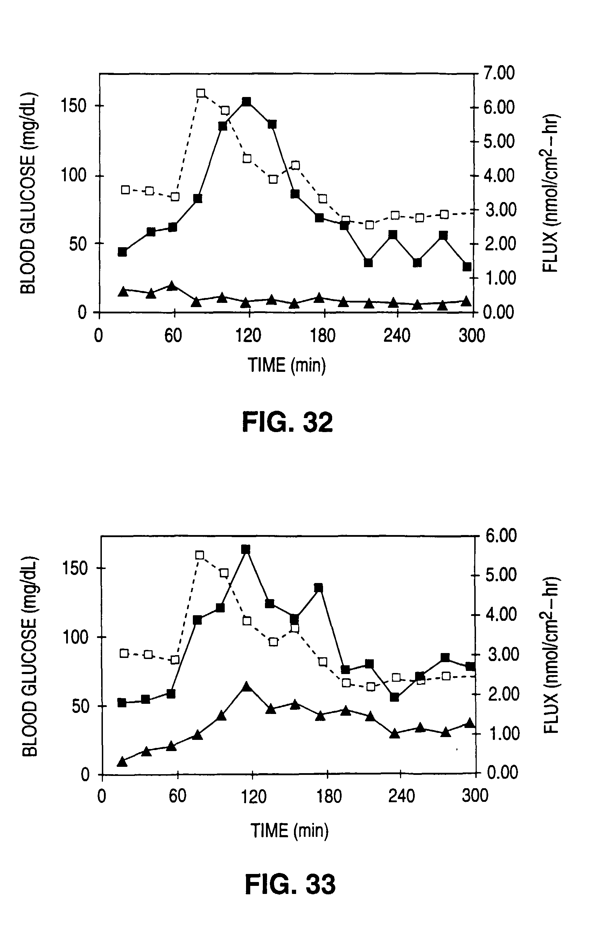

Two iontophoretic extractions using a modified buffer solution were performed simultaneously on the same subject (A) as in Figures 17 and 18, one extraction using the standard (DC) protocol on the right arm and the other using the alternating polarity protocol on the left arm. Iontophoresis was administered and glucose was monitored as described in Example 4 above, with the following modification. Sodium bicarbonate buffer solution, 5% U.S.P., pH 7-9, mixed in a 1:9 ration with 0.45% sodium chloride U.S.P. was used as the collector solution instead of 0.45% sodium chloride. The results are set forth in Figures 32 and 33.

Figure 32 demonstrated the dramatic enhancement in flux at the cathode for a direct current protocol using pH 8.2 sodium bicarbonate differed saline solution compared to Figure 17 for the same subject (6) using 0.45% sodium chloride solution.

Figure 33 demonstrated the dramatic enhancement of flux at the cathode for the alternating polarity protocol using pH 8.2 sodium bicarbonate buffered saline solution compared to Figure 18 for the same subject (6) using 0.45% sodium chloride solution.

Further, a comparison of Figures 32 and 33 demonstrated that there was enhanced anode extraction for the sodium bicarbonate solution in the alternating polarity method of the direct current method.

## Claims

1. An iontophoresis apparatus for the transdermal monitoring of a target substance comprising:
a collection reservoir, the collection reservoir comprising either an ionically conductive hydrogel or a wicking material and an ionically conductive solution;
first and second iontophoresis electrodes;
an iontophoretic power source;
characterised in that it also comprises a sensor for the target substance, wherein the sensor is in contact with the collection reservoir.

2. The apparatus of claim 1 wherein the reservoir comprises an ionically conductive hydrogel.

3. The apparatus of claim 1 wherein the reservoir comprises a wicking material and an ionically conductive solution.

4. The apparatus of claim 1 further comprising a second reservoir, the second reservoir comprising either a second ionically conductive hydrogel or wicking material and an ionically conductive solution.

5. The apparatus of claim 4 further comprising a housing and means for separably holding the first and second reservoirs within the housing in contact with the first and second iontophoresis electrodes, respectively.

6. The apparatus of claim 1 wherein the sensor comprises a sensor electrode assembly.

7. The apparatus of claim 1 wherein the sensor comprises a plurality of sensor electrodes, the sensor electrodes and the first iontophoresis electrode being disposed in substantially the same plane.

8. The apparatus of claim 7 wherein the reservoir has an electrode contact surface, the sensor electrodes and the first iontophoresis electrode being adapted to contact the electrode contact surface.

9. The apparatus of claim 6 wherein the sensor comprises an enzyme within the reservoir.

10. The apparatus of claim 6 further comprising a display for displaying target substance information.

11. The apparatus of claim 1 comprising:
first and second collection reservoirs, the first collection reservoir comprising a first ionically conductive hydrogel and the second collection reservoir comprising a second ionically conductive hydrogel;
wherein the first iontophoresis electrode is in contact with the first hydrogel and the second iontophoresis electrode is in contact with the second hydrogel; and
wherein the sensor for detecting the target substance is contained within at least one hydrogel.

12. The apparatus of claim 11 wherein the sensor comprises a first sensor electrode assembly in contact with the first hydrogel and a second sensor electrode assembly in contact with the second hydrogel.

13. The apparatus of claim 12 further comprising a housing containing the collection reservoirs, the iontophoresis electrodes, the sensor electrode assemblies and the power source.

14. The apparatus of claim 12 further comprising means for separably holding the first and second hydrogels in contact with the iontophoresis electrodes and the sensor electrode assemblies.

15. The apparatus of claim 14 wherein the first and second hydrogels each have an electrode contact surface, the sensor electrode assemblies and the first iontophoresis electrode being adapted to contact their respective electrode contact surface.

16. The apparatus of claim 15 wherein the means for separably holding comprises an electrode board in which the iontophoresis electrodes and the sensor electrode assemblies are disposed.

17. The apparatus of claim 11 further comprising a display for displaying target substance information.

18. The apparatus of claim 17 further comprising a housing containing the collection reservoirs, the iontophoresis electrodes, the sensor electrode assemblies, the power source and the display.

19. The apparatus of claim 1, wherein the collection reservoir comprises an ionically conductive hydrogel having a pH in the range of from about 4.0 to about 10.0 and an enzyme reactive with the target substance.

20. The collection reservoir of claim 19 wherein the enzyme is glucose oxidase.

21. The collection reservoir of claim 20 wherein the hydrogel has a hydrogen peroxide concentration less than 80 µM.

22. The collection reservoir of claim 19 wherein the hydrogel further comprises an electrode contact surface adapted to contact an iontophoresis electrode.

23. The collection reservoir of claim 22 wherein the electrode contact surface is further adapted to contact a sensor electrode.

24. The collection reservoir of claim 23 wherein the hydrogel has a substantially cylindrical shape, the electrode contact surface being a circular surface at one end of the hydrogel cylinder.

25. The apparatus of claim 1 wherein the target substance is glucose thus providing a glucose monitor wherein
first and second glucose collection reservoirs each contain glucose collection medium selected from a group consisting of water and saline solution;
the power supply has a positive connector and a negative connector;
conductors and a switch electrically connect the first and second collection reservoirs to the power supply positive connector and the power supply negative connector, the switch having a first position in which the first collection reservoir is electrically connected to the positive connector and the second collection reservoir is electrically connected to the negative connector and a second position in which the second collection reservoir is electrically connected to the positive connector and the first collection reservoir is electrically connected to the negative connector.

26. The apparatus of claim 25 wherein the first and second electrodes are in electrical communication with the collection medium of the first and second collection reservoirs, respectively, and with the power supply through a conductor and the switch.

27. The apparatus of claim 26 further comprising a collection medium glucose concentration analyzer and calibration means for correlating collection medium glucose concentration with blood glucose concentration.

28. The apparatus of claim 25 further comprising a collection medium glucose concentration analyzer and calibration means for correlating collection medium glucose concentration with blood glucose concentration.

29. The apparatus of claim 25 in which the switch is a manual switch.

30. The apparatus of claim 25 in which the switch is an automatic programmable switch.

## Patentansprüche

1. Iontophoreseapparat zum transdermalen Anzeigen bzw. Messen bzw. Überwachen einer Zielsubstanz mit:
einem Sammelreservoir bzw. Sammelbehälter, wobei das Sammelreservoir entweder ein ionisch leitendes Hydrogel oder ein Dochtmaterial bzw. geflochtenes Material bzw. gewirktes Material bzw. Wirkmaterial und eine ionisch leitende Lösung aufweist;
eine erste und eine zweite lontophoreseelektrode;
eine iontophoresische Leitungs- bzw. Stromquelle;
dadurch gekennzeichnet,
daß der Apparat auch einen Sensor für die Zielsubstanz enthält, wobei der Sensor in Kontakt mit dem Sammelreservoir steht.

2. Apparat nach Anspruch 1, wobei das Reservoir ein ionisch leitendes Hydrogel aufweist.

3. Apparat nach Anspruch 1, wobei das Reservoir ein Wirkmaterial und eine ionisch leitende Lösung aufweist.

4. Apparat nach Anspruch 1 weiterhin aufweisend ein zweites Reservoir, wobei das zweite Reservoir entweder ein zweites ionisch leitendes Hydrogel oder Wirkmaterial und eine ionisch leitende Lösung aufweist.

5. Apparat nach Anspruch 4 weiterhin aufweisend ein Gehäuse und Mittel zum trennbaren Halten des ersten und zweiten Reservoirs innerhalb des Gehäuses in Kontakt mit der ersten bzw. zweiten lontophoreseelektrode.

6. Apparat nach Anspruch 1, wobei der Sensor eine Sensorelektrodenanordnung aufweist.

7. Apparat nach Anspruch 1, wobei der Sensor eine Vielzahl von Sensorelektroden aufweist, wobei die Sensorelektroden und die erste lontophoreseelektrode im wesentlichen in der gleichen Ebene angordnet sind.

8. Apparat nach Anspruch 7, wobei das Reservoir eine Elektrodenkontaktfläche hat, wobei die Sensorelektroden und die erste lontophoreseelektrode angepaßt sind, um die Elektrodenkontaktfläche zu kontaktieren.

9. Apparat nach Anspruch 6, wobei der Sensor ein Enzym innerhalb des Reservoir aufweist.

10. Apparat nach Anspruch 6 weiterhin aufweisend eine Anzeige bzw. Display zum Anzeigen von Zielsubstanzinformation.

11. Apparat nach Anspruch 1 mit:
erstem und zweitem Sammelreservoir, wobei das erste Sammelreservoir ein erstes ionisch leitendes Hydrogel aufweist und wobei das zweite Sammelreservoir ein zweites ionisch leitendes Hydrogel aufweist;
wobei die erste lontophoreseelektrode in Kontakt mit dem ersten Hydrogel ist und die zweite lontophoreseelektrode in Kontakt mit dem zweiten Hydrogel ist und
wobei der Sensor zum Erfassen der Zielsubstanz in mindestens einem Hydrogel enthalten ist.

12. Apparat nach Anspruch 11, wobei der Sensor eine erste Sensorelektrodenanordnung in Kontakt mit dem ersten Hydrogel und eine zweite Sensorelektrodenanordnung in Kontakt mit dem zweiten Hydrogel aufweist.

13. Apparat nach Anspruch 12 weiterhin aufweisend ein Gehäuse, das die Sammelreservoirs, die lontophoresenelektroden, die Sensorelektrodenanordnungen und die Stromquelle enthält.

14. Apparat nach Anspruch 12 weiterhin aufweisend Mittel zum trennbaren Halten des ersten und zweiten Hydrogels in Kontakt mit den lontophoresenelektroden und den Sensorelektrodenanordnungen.

15. Apparat nach Anspruch 14, wobei das erste und zweite Hydrogel jeweils eine Elektrodenkontaktfläche haben, die Sensorelektrodenanordnungen und die erste lontophoreseelektrode angepaßt sind, um deren jeweilige Elektrodenkontaktfläche zu kontaktieren.

16. Apparat nach Anspruch 15, wobei die Mittel zum trennbaren Halten eine Elektrodenplatte bzw. -platine bzw. -tafel aufweisen, an welcher die lontophoresenelektroden und die Sensorelektrodenanordnung angeordnet sind.

17. Apparat nach Anspruch 11 weiterhin aufweisend eine Anzeige zum Anzeigen von Zielsubstanzinformation.

18. Apparat nach Anspruch 17 weiterhin aufweisend ein Gehäuse, das die Sammelreservoirs, die lontophoresenelektroden, die Sensorelektrodenanordnungen, die Stromquelle und die Anzeige aufweist.

19. Apparat nach Anspruch 1, wobei das Sammelreservoir ein ionisch leitendes Hydrogel aufweist, das einen pH-Wert im Bereich von 4,0 bis etwa 10,0 und ein Enzym aufweist, das mit der Zielsubstanz reagiert.

20. Apparat nach Anspruch 19, wobei das Enzym Glukoseoxydase ist.

21. Apparat nach Anspruch 20, wobei das Hydrogel eine Wasserstoffperoxidkonzentration von weniger als 80 µM aufweist.

22. Apparat nach Anspruch 19, wobei das Hydrogel weiter eine angepaßte Elektrodenkontaktfläche aufweist, um eine lontophoreseelektrode zu kontaktieren.

23. Apparat nach Anspruch 22, wobei die Elektrodenkontaktfläche weiter angepaßt ist, um eine Sensorelektrode zu kontaktieren.

24. Apparat nach Anspruch 23, wobei das Hydrogel eine im wesentlichen zylindrische Form hat, die Elektrodenkontaktfläche, eine kreisförmige Fläche an einem Ende des Hydrogelzylinders ist.

25. Apparat nach Anspruch 1, wobei
die Zielsubstanz Glukose ist und somit eine Glukosemeßeinrichtung bzw. Glukoseüberwachungseinrichtung bzw. Glukose-Monitor bereitstellt,
ein erstes und zweites Glukosesammelreservoir jeweils ein Glukosesammelmedium enthalten, ausgewählt aus einer Gruppe bestehend aus einer Wasser- und einer Salz- bzw. Kochsalzlösung;
die Stromquelle einen positiven und einen negativen Anschluß hat;
Drähte bzw. Leiter und ein Schalter die ersten und zweiten Sammelreservoirs mit dem positiven Anschluß der Stromquelle und dem negativen Anschluß der Stromquelle elektrisch verbinden, wobei der Schalter eine erste Position aufweist, in welcher das erste Sammelreservoir elektrisch mit dem positiven Anschluß verbunden ist und das zweite Sammelreservoir elektrisch mit dem negativen Anschluß verbunden ist und eine zweite Position, in welcher das zweite Sammelbecken elektrisch mit dem positiven Anschluß verbunden ist und das erste Sammelbecken elektrisch mit dem negativen Anschluß verbunden ist.

26. Apparat nach Anspruch 25, wobei die erste und zweite Elektrode mit dem Sammelmedium des ersten bzw. zweiten Sammelreservoirs und mit der Stromquelle durch einen Leiter und dem Schalter elektrisch verbunden sind.

27. Apparat nach Anspruchs 26 aufweisend einen Analysierer die Sammelmediumglukosekonzentration und ein Kalibrierungsmittel, um die Sammelmediumglukosekonzentration mit der Blutglukosekonzentration zu korrelieren.

28. Apparat nach Anspruchs 25 weiterhin aufweisend einen Analysierer für die Sammelmediumglukosekonzentration und ein Kalibrierungsmittel, um die Sammelmediumglukosekonzentration mit der Blutglukosekonzentration zu korrelieren.

29. Apparat nach Anspruch 25, in welchem der Schalter ein manueller Schalter ist.

30. Apparat nach Anspruch 25, in welchem der Schalter ein automatischer progammierbarer Schalter ist.

## Revendications

1. Appareil d'iontophorèse pour le contrôle transdermique d'une substance cible comprenant:
un réservoir collecteur, le réservoir collecteur comprenant soit un hydrogel conducteur ionique soit un matériau à effet de mèche et une solution conductrice ionique;
une première et une seconde électrode d'iontophorèse;
une source d'énergie iontophorétique; caractérisé en ce qu'il comprend également un détecteur pour la substance cible, le détecteur étant en contact avec le réservoir collecteur.

2. Appareil selon la revendication 1, dans lequel le réservoir contient un hydrogel conducteur ionique.

3. Appareil selon la revendication 1, dans lequel le réservoir contient un matériau à effet de mèche et une solution conductrice ionique.

4. Appareil selon la revendication 1, comprenant, en outre, un second réservoir, le second réservoir comprenant soit un second hydrogel conducteur ionique soit un matériau à effet de mèche et une solution conductrice ionique.

5. Appareil selon la revendication 4, comprenant, en outre, un boîtier et un moyen pour maintenir de façon séparable le premier et le second réservoir dans le boîtier, respectivement au contact de la première et de la seconde électrode d'iontophorèse.

6. Appareil selon la revendication 1, dans lequel le détecteur comprend un ensemble d'électrodes détectrices.

7. Appareil selon la revendication 1, dans lequel le détecteur comprend plusieurs électrodes détectrices, les électrodes détectrices et la première électrode d'iontophorèse étant disposées sensiblement dans le même plan.

8. Appareil selon la revendication 7, dans lequel le réservoir possède une surface de contact pour les électrodes, les électrodes détectrices et la première électrode d'iontophorèse étant adaptées pour entrer en contact avec la surface de contact pour les électrodes.

9. Appareil selon la revendication 6, dans lequel le détecteur comprend une enzyme à l'intérieur du réservoir.

10. Appareil selon la revendication 6, comprenant, en outre, une unité d'affichage pour afficher les informations concernant la substance cible.

11. Appareil selon la revendication 1 comprenant :
un premier et un second réservoir collecteur, le premier réservoir collecteur contenant un premier hydrogel conducteur ionique et le second réservoir collecteur contenant un second hydrogel conducteur ionique;
dans lequel la première électrode d'iontophorèse est en contact avec le premier hydrogel et la seconde électrode d'iontophorèse est en contact avec le second hydrogel; et
dans lequel le détecteur servant à détecter la substance cible est placé dans au moins un hydrogel.

12. Appareil selon la revendication 11, dans lequel le détecteur comprend un premier ensemble d'électrodes détectrices en contact avec le premier hydrogel et un second ensemble d'électrodes détectrices en contact avec le second hydrogel.

13. Appareil selon la revendication 12, comprenant, en outre, un boîtier contenant les réservoirs collecteurs, les électrodes d'iontophorèse, les ensembles d'électrodes détectrices et la source d'énergie.

14. Appareil selon la revendication 12, comprenant, en outre, un moyen pour maintenir de façon séparable le premier et le second hydrogel au contact des électrodes d'iontophorèse et des ensembles d'électrodes détectrices.

15. Appareil selon la revendication 14, dans lequel le premier et le second hydrogel possèdent chacun une surface de contact pour les électrodes, les ensembles d'électrodes détectrices et la première électrode d'iontophorèse étant adaptés pour entrer en contact avec leur surface de contact respective pour électrodes.

16. Appareil selon la revendication 15, dans lequel le moyen de maintien séparable comprend une plaque d'électrode dans laquelle sont disposés les électrodes d'iontophorèse et les ensembles d'électrodes détectrices.

17. Appareil selon la revendication 11, comprenant, en outre, une unité d'affichage pour afficher les informations concernant la substance cible.

18. Appareil selon la revendication 17, comprenant, en outre, un boîtier contenant les réservoirs collecteurs, les électrodes d'iontophorèse, les ensembles d'électrodes détectrices, la source d'énergie et l'unité d'affichage.

19. Appareil selon la revendication 1, dans lequel le réservoir collecteur comprend un hydrogel conducteur ionique ayant un pH compris dans la gamme d'environ 4,0 à environ 10,0 et une enzyme réactive avec la substance cible.

20. Réservoir collecteur selon la revendication 19, dans lequel l'enzyme est la glucose oxydase.

21. Réservoir collecteur selon la revendication 20, dans lequel l'hydrogel a une concentration en peroxyde d'hydrogène inférieure à 80 µM.

22. Réservoir collecteur selon la revendication 19, dans lequel l'hydrogel comprend en outre une surface de contact pour les électrodes qui est adaptée pour entrer en contact avec une électrode d'iontophorèse.

23. Réservoir collecteur selon la revendication 22, dans lequel la surface de contact pour électrodes est, en outre, adaptée pour enter en contact avec une électrode détectrice.

24. Réservoir collecteur selon la revendication 23, dans lequel l'hydrogel a une forme sensiblement cylindrique, la surface de contact pour les électrodes étant une surface circulaire à l'une des extrémités du cylindre de l'hydrogel.

25. Appareil selon la revendication 1, dans lequel la substance cible est le glucose, fournissant ainsi un appareil de contrôle du glucose dans lequel:
le premier et le second réservoir collecteur de glucose contiennent chacun un milieu de recueil de glucose choisi dans un groupe constitué par l'eau et une solution saline;
l'alimentation électrique possède un connecteur positif et un connecteur négatif;
des conducteurs et un commutateur relient électriquement le premier et le second réservoir collecteur au connecteur positif d'alimentation électrique et au connecteur négatif d'alimentation électrique, le commutateur ayant une première position dans laquelle le premier réservoir collecteur est relié électriquement au connecteur positif et le second réservoir collecteur est relié électriquement au connecteur négatif, et une deuxième position dans laquelle le second réservoir collecteur est relié électriquement au connecteur positif et le premier réservoir collecteur est relié électriquement au connecteur négatif.

26. Appareil selon la revendication 25, dans lequel la première et la seconde électrode sont en communication électrique avec le milieu de recueil du premier et du second réservoir collecteur, respectivement, et avec l'alimentation électrique par l'intermédiaire d'un conducteur et du commutateur.

27. Appareil selon la revendication 26, comprenant, en outre, un analyseur de concentration de glucose du milieu de recueil et un moyen d'étalonnage pour corréler la concentration de glucose du milieu de recueil avec la concentration de glucose dans le sang.

28. Appareil selon la revendication 25, comprenant, en outre, un analyseur de concentration de glucose du milieu de recueil et un moyen d'étalonnage pour corréler la concentration de glucose du milieu de recueil avec la concentration de glucose dans le sang.

29. Appareil selon la revendication 25, dans lequel le commutateur est un commutateur manuel.

30. Appareil selon la revendication 25, dans lequel le commutateur est un commutateur automatique programmable.
